# EUROPEAN PATENT SPECIFICATION

(11) **EP 1 857 453 B1**
(45) Date of publication and mention of the grant of the patent: **29.12.2010**
(21) Application number: 06712109.5
(22) Date of filing: 20.01.2006
(51) Int. Cl.: C07D 401/04, A61K 31/4709, A61P 31/04

(54) **FLUOROALKYLPYRROLIDINE DERIVATIVES**
FLUOROALKYLPYRROLIDIN-DERIVATE
DERIVES DE FLUOROALKYLPYRROLIDINE

(30) Priority: 21.01.2005 JP 2005013738
(43) Date of publication of application: 21.11.2007
(73) Proprietor: Daiichi Sankyo Company, Limited, Chuo-ku Tokyo 103-8426 (JP)
(72) Inventor: TAKAHASHI, Hisashi, Daiichi Sankyo Company, Ltd, Edogawa-ku, Tokyo 134-8630 (JP); MIYAUCHI, Rie, Daiichi Sankyo Company, Ltd., Edogawa-ku, Tokyo 134-8630 (JP); TAKEMURA, Makoto, Daiichi Sankyo Company, Ltd., Edogawa-ku, Tokyo 134-8630 (JP)
(74) Representative: Hartz, Nikolai
(86) International application number: PCT/JP2006/300884
(87) International publication number: WO 2006/077984

(56) References cited:
- EP-A- 0 237 955
- EP-A- 0 995 744
- JP-A- 02 019 377
- JP-A- 62 215 572
- KAWAKAMI K ET AL: "Studies on 8-methoxyquinolones: synthesis and antibacterial activity of 7-(3-amino-4-substituted)pyrrolidinyl derivatives" CHEMICAL AND PHARMACEUTICAL BULLETIN, PHARMACEUTICAL SOCIETY OF JAPAN, TOKYO, vol. 48, no. 11, 1 November 2000 (2000-11-01), pages 1667-1672, XP002989843 ISSN: 0009-2363
- KAWAKAMI K. ET AL.: 'Studies on 8-methoxyquinolones: Synthesis and antibacterial activity of 7-(3-amino-4-substituted)pyrrolidinyl derivatives' CHEMICAL PHARMACEUTICAL BULLETIN vol. 48, no. 11, 2000, pages 1667 - 1672, XP002989843

## Description

### TECHNICAL FIELD

This invention relates to a fluoroalkylpyrrolidine derivative which exhibits excellent antibacterial activity for Gram positive and Gram negative bacteria, and a drug containing the same as an effective component.

### BACKGROUND ART

Since discovery of norfloxacin, quinolone synthetic antibacterial drugs have been considerably improved in respect of antibacterial activity and pharmacokinetics, and made great progress to be used in chemotherapy for infections including systemic infections, and many compounds are in clinical use.
However, bacteria with low sensitivity to quinolone synthetic antibacterial drugs have increasingly come to be observed in clinical fields. For example, bacteria which are resistant to drugs other than quinolone synthetic antibacterial drugs and have low sensitivity to quinolone synthetic antibacterial drugs are on the increase, as can be seen in the case of Gram positive bacteria, such as Gram positive coccus like Staphylococcus aureus (MRSA) and pneumococcus (PRSP) insensitive to β-lactam antibiotics and enterococcus (VRE) insensitive to aminoglycoside antibacterial drugs.
Accordingly, there is strong demand for a drug much more effective against Gram-positive coccus, especially from the clinical standpoint.
There is also demand for development of quinolone synthetic antibacterial drugs with improved safety, since using such drug in combination with nonsteroidal antiinflammatory drugs (NSAIDs) is known to induce side effects such as convulsion, central action (mild central neuropathy such as shakiness, headache, and insomnia, as well as serious side effects such as convulsion), phototoxicity (photosensitivity), hepatotoxicity, cardiotoxicity (abnormality observed by abnormal electrocardiogram which may induce fatal arrhythmia), and abnormal blood glucose level (See Non-patent Documents 1 and 2).

In the meanwhile, the structure of the substituent at position 7 of the quinolone skeleton is known to have a great deal of influence on the antibacterial activity, pharmacokinetics, and safety of the quinolone synthetic antibacterial drugs. For example, quinolone derivatives having 3-aminopyrrolidine-1-yl group as a substituent are known to have more favorable antibacterial activity to Gram negative and Gram positive bacteria, compared to the quinolone derivatives having piperazine derivative as the substituent (See Non-patent Documents 3 and 4).
Despite the strong antibacterial activity, many of the quinolone derivatives having 3-aminopyrrolidine-1-yl group as the substituent have stronger cytotoxicity and micronucleus induction in erythrocyte cells and lower selective toxicity, compared to the quinolone derivatives having piperazine derivative as the substituent (see Non-patent Document 4). Such quinolone derivatives also affect eukaryotic cells and therefore is difficult to use as drugs for humans and animals. Hence, a drug designed to have an improved selective toxicity is in great need.
Accordingly, there has been growing demand for development of a compound having both strong antibacterial activity and high selective toxicity, from the clinical standpoint.

Patent Document 1 and Non-patent Document 5 disclose a quinolone carboxylic acid derivative (A) having a cis-3-amino-4-(fluorine substituted methyl)pyrrolidine-1-yl group as a substituent at position 7. (Please note that the definitions of the substituents for the formula (A) are taken from Patent Document 1, and a symbol which happens to be the same as the one used in the present invention may designate a substituent different from the one defined in the present invention.)

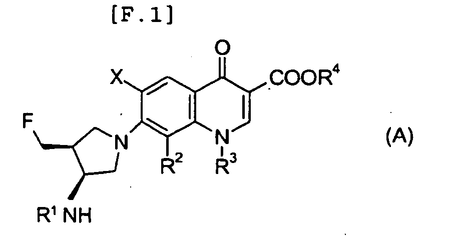

The substituent at position 8 of the quinolone skeleton (corresponding to group R²) is limited to a halogenomethoxy group and an alkoxy group, and there is no definite indication of a quinolone carboxylic acid derivative wherein the substituent at position 7 of the quinolone skeleton is a cis-3-amino-4-(fluorine substituted methyl)pyrrolidine-1-yl group and the substituent at position 8 is an alkyl group or a halogen-substituted alkyl group.

Non-patent Document 6 discloses a quinolone carboxylic acid derivative wherein the substituent at position 7 is a cis-3-amino-4-(fluorine substituted methyl)pyrrolidine-1-yl group, and exemplary compounds disclosed therein include 8-methoxy quinolone derivative (B) having cis-3-amino-4-trifluoromethylpyrrolidine-1-yl group as its substituent.

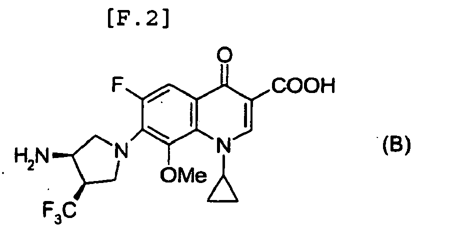

However, in the compound described in Non-patent Document 6, the substituent at position 8 of the quinolone skeleton is limited to methoxy group, and there is no particular indication of the quinolone carboxylic acid derivative wherein the substituent at position 7 of the quinolone skeleton is a cis-3-amino-4-(fluorine substituted methyl)pyrrolidine-1-yl group and the substituent at position 8 is an alkyl group or a halogen-substituted alkyl group.

Non-patent Document 7 discloses a quinolone carboxylic acid derivative wherein the substituent at position 7 is a cis-3-amino-4-(fluorine substituted methyl)pyrrolidine-1-yl group, and exemplary compounds disclosed therein include 2-pyridone derivative (9-methyl-4H-4-oxoquinolizine-3-carboxylic acid derivative) (C) having cis-3-amino-4-trifluoromethylpyrrolidine-1-yl group as the substituent.

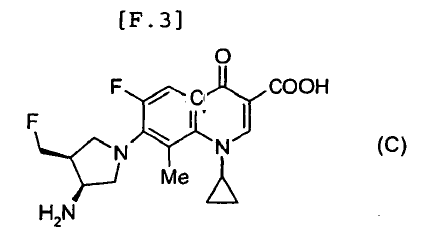

However, in the compound described in Non-patent Document 7, the quinolone skeleton is limited to 2-pyridone derivative (9-methyl-4H-4-oxoquinolizine-3-carboxylic acid derivative), which is different in chemical structure from the 1,4-dihydro-8-methyl-4-oxoquinoline-3-carboxylic acid derivative within the scope of the present invention.
[Patent Document 1] WO98/58923
[Non-patent Document 1] Hiroyuki Kobayashi Ed., "Clinical Applications of New-quinolone Agents", Iyaku-Journal-Sha (2001)
[Non-patent Document 2] Drugs, Vol. 62, No. 1, page 13 (2002)
[Non-patent Document 3] International Journal of Antimicrobial Agents, Vol. 16, page 5 (2000)
[Non-patent Document 4] Journal of Antimicrobial Chemotherapy, Vol. 33, page 685 (1994)
[Non-patent Document 5] Chemical and Pharmaceutical Bulletin, Vol. 48 (No. 11), page 1667 (2000)
[Non-patent Document 6] Bioorganic Medicinal Chemistry Letters, Vol. 8, page 2833 (1998)
[Non-patent Document 7] Bioorganic Medicinal Chemistry Letters, Vol. 8, page 1953 (1998)

### DISCLOSURE OF THE INVENTION

### PROBLEMS TO BE SOLVED BY THE INVENTION

In view of the situation as described above, an object of the present invention is to provide a quinolone antibacterial drug and a prophylactic and/or therapeutic drug for an infection which exhibit broad and strong antibacterial activity to both Gram positive and Gram negative bacteria, and which are also highly safe.

### MEANS FOR SOLVING THE PROBLEMS

The present inventors found that the compound represented by the following formula (1) has broad and strong antibacterial activity to both Gram positive and Gram negative bacteria, and that such compound is also highly safe in use for an antibacterial drug or a prophylactic and/or therapeutic drug for an infection. The present invention has been completed on the basis of such findings.

Accordingly, this invention provides a compound represented by the following formula (1):

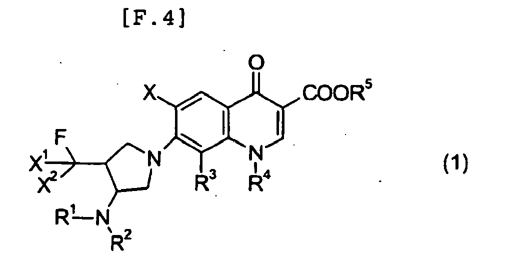

or its salt, or a hydrate thereof, wherein
R¹ represents hydrogen atom, an alkyl group containing 1 to 6 carbon atoms, a cycloalkyl group containing 3 to 6 carbon atoms, or a substituted carbonyl group derived from an amino acid, a dipeptide, or a tripeptide, wherein the alkyl group is optionally substituted with a group selected from the group consisting of hydroxy group, amino group, halogen atom, alkylthio group containing 1 to 6 carbon atoms, and alkoxy group containing 1 to 6 carbon atoms;
R² represents hydrogen atom, an alkyl group containing 1 to 6 carbon atoms, or a cycloalkyl group containing 3 to 6 carbon atoms, wherein the alkyl group is optionally substituted with a group selected from the group consisting of hydroxy group, amino group, halogen atom, alkylthio group containing 1 to 6 carbon atoms, and alkoxy group containing 1 to 6 carbon atoms;
R³ represents an alkyl group containing 1 to 6 carbon atoms;
R⁴ represents a cycloalkyl group containing 3 to 6 carbon atoms or a halogen-substituted cycloalkyl group containing 3 to 6 carbon atoms;
R⁵ represents hydrogen atom, phenyl group, acetoxymethyl group, pivaloyloxymethyl group, ethoxycarbonyl group, choline group, dimethylaminoethyl group, 5-indanyl group, phthalidyl group, 5-alkyl-2-oxo-1,3-dioxol-4-ylmethyl group, 3-acetoxy-2-oxobutyl group, an alkyl group containing 1 to 6 carbon atoms, an alkoxymethyl group containing 2 to 7 carbon atoms, or a phenylalkyl group comprising an alkylene group containing 1 to 6 carbon atoms and phenyl group;
X¹ and X² independently represent hydrogen atom or a halogen atom; and
X represents hydrogen atom or a halogen atom.

This invention also provides 7-[(3S,4S)-3-amino-4-fluoromethylpyrrolidine-1-yl]-6-fluoro-1-[(2S,1R)-2-fluorocyclopropyl]-1,4-dihydro-8-methyl-4-oxoquinoline-3-carboxylic acid, or its salt or a hydrate thereof; and 7-[(3S,4S)-3-fluoromethyl-4-methylaminopyrrolidine-1-yl]-6-fluoro-1-[(2S,1R)-2-fluorocyclopropyl]-1,4-dihydro-8-methyl-4-oxoquinoline-3-carboxylic acid, its salt, or a hydrate thereof.
This invention also provides a drug containing the compound represented by the formula (1), its salt or a hydrate thereof as an effective component.
This invention also provides a pharmaceutical composition containing the compound represented by the formula (1), its salt or a hydrate thereof, and a pharmaceutically acceptable carrier.
This invention also provides a method for treating a disease, which comprises administering an effective amount of the compound represented by the formula (1), its salt or a hydrate thereof.
This invention also provides a method for producing a drug, which comprises blending the compound represented by the formula (1), its salt or a hydrate thereof, as an effective component in the drug.
This invention also provides use of the compound represented by the formula (1), its salt or a hydrate thereof for the production of a drug.

### ADVANTAGEOUS EFFECT OF THE INVENTION

The fluoroalkylpyrrolidine derivative of the present invention has excellent antibacterial activity for both Gram positive and Gram negative bacteria as well high safety with weak acute toxicity. Accordingly, the fluoroalkylpyrrolidine derivative of the present invention is useful as an antibacterial drug or as a prophylactic and/or therapeutic drug for an infection.

### BEST MODES FOR CARRYING OUT THE INVENTION

Next, the substituents of the compound of the present invention represented by the formula (1) are described.

Substituent R¹ represents hydrogen atom, an alkyl group containing 1 to 6 carbon atoms, a cycloalkyl group containing 3 to 6 carbon atoms, or a substituted carbonyl group derived from an amino acid, a dipeptide, or a tripeptide.
Substituent R² may be hydrogen atom, an alkyl group containing 1 to 6 carbon atoms, or a cycloalkyl group containing 3 to 6 carbon atoms.
When R¹ or R² is an alkyl group, it may have a substituent selected from the group consisting of hydroxy group, amino group, halogen atom, alkylthio group containing 1 to 6 carbon atoms, and alkoxy group containing 1 to 6 carbon atoms.

When R¹ or R² is an alkyl group, they may be a straight chain alkyl group such as methyl group, ethyl group, n-propyl group, n-butyl group, or n-pentyl group; or a branched alkyl group such as isopropyl group, isobutyl group, sec-butyl group, or tert-butyl group. Among these, the preferred are methyl group and ethyl group, and the more preferred is methyl group.
When such alkyl group has hydroxy group or amino group as its substituent, the hydroxy group or the amino group is preferably a substituent on the terminal carbon atom of the alkyl group. The alkyl group in the alkyl group having the hydroxy group is preferably an alkyl group containing up to 3 carbon atoms, and preferable exemplary alkyl groups having the hydroxy group include hydroxymethyl group, 2-hydroxyethyl group, 2-hydroxypropyl group, and 3-hydroxypropyl group. The alkyl group in the alkyl group having the amino group is preferably an alkyl group containing up to 3 carbon atoms, and preferable exemplary alkyl groups having the amino group include aminomethyl group, 2-aminoethyl group, 2-aminopropyl group, and 3-aminopropyl group.

When such alkyl group has a halogen atom as its substituent, the alkyl group may be a straight chain or a branched alkyl group containing 1 to 6 carbon atoms. Exemplary halogen atoms include fluorine atom, chlorine atom, and iodine atom, and the preferred is fluorine atom. The alkyl group may be mono-, di-, or trisubstituted by fluorine atoms, and the examples include monofluoromethyl group, difluoromethyl group, trifluoromethyl group, and 2,2,2-trifluoroethyl group.

When such alkyl group has an alkylthio group or an alkoxy group as its substituent, the alkyl group may be a straight chain or a branched alkyl group containing 1 to 6 carbon atoms, and the alkyl moiety in the alkylthio group or the alkoxy group may also be a straight chain or a branched moiety. The alkyl group having an alkylthio group is preferably an alkylthiomethyl group, an alkylthioethyl group, or an alkylthiopropyl group, and the alkylthio group is preferably the one having 1 to 3 carbon atoms. Preferable examples of the alkyl group having an alkylthio group include methylthiomethyl group, ethylthiomethyl group, and methylthioethyl group. The alkyl group having an alkoxy group is preferably an alkoxymethyl group, an alkoxyethyl group, or an alkoxypropyl group, and the alkoxy group is preferably the one having 1 to 3 carbon atoms. Preferable examples of the alkyl group having an alkoxy group include methoxymethyl group, ethoxymethyl group, and methoxyethyl group.

When R¹ or R² is a cycloalkyl group, it is preferably cyclopropyl group or cyclobutyl group, and more preferably cyclopropyl group.

Preferable combinations of R¹ and R² include combinations of R¹ which is hydrogen atom, an alkyl group, a cycloalkyl group, or a substituted carbonyl group derived from an amino acid, a dipeptide, or a tripeptide with R² which is hydrogen atom. Among these, the preferred are those wherein R¹ is hydrogen atom, an alkyl group, or a cycloalkyl group and R² is hydrogen atom. In this case, the alkyl group is preferably methyl group or ethyl group, and most preferably, methyl group. The cycloalkyl group is preferably cyclopropyl group or cyclobutyl group, and most preferably, cyclopropyl group. More preferable combinations are the combination wherein both R¹ and R² are hydrogen, and the combination wherein R¹ is methyl group and R² is hydrogen atom.

A quinolone derivative wherein the substituent R¹ is a substituted carbonyl group derived from an amino acid, a dipeptide, or a tripeptide and the substituent R² is hydrogen atom is particularly preferable for use as a prodrug.

The amino acid, the dipeptide, or the tripeptide used for producing such prodrug may be the one which produces free amine compound upon cleavage of the amide bond between the carboxyl group and the nitrogen atom of the amino group at position 3 of the pyrrolidine ring in a living body. Exemplary such substituents include substituted carbonyl groups derived from an amino acid such as glycine, alanine, or aspartic acid; a dipeptide such as glycine-glycine, glycine-alanine, or alanine-alanine; or a tripeptide such as glycine-glycine-alanine, or glycine-alanine-alanine.

Substituent R³ represents an alkyl group containing 1 to 6 carbon atoms.
Exemplary an alkyl groups containing 1 to 6 carbon atoms include those mentioned above. Among these, the preferred is an alkyl group containing 1 to 3 carbon atoms, and the most preferred is methyl group. Exemplary halogen groups contained in the halogen-substituted alkyl group containing 1 to 6 carbon atoms include fluorine atom and chlorine atom, and the number of the halogen atom is preferably 1 to 3.

Substituent R⁴ represents a cycloalkyl group containing 3 to 6 carbon atoms or a halogen-substituted cycloalkyl group containing 3 to 6 carbon atoms.
Exemplary cycloalkyl groups containing 3 to 6 carbon atoms include those as mentioned above. Among these, the preferred is.cyclopropyl group. Exemplary halogen-substituted cycloalkyl groups containing 3 to 6 carbon atoms include cycloalkyl groups as mentioned above substituted with 1 or 2 halogen atom. Exemplary halogen atoms include fluorine atom and chlorine atom, and the preferred is fluorine atom. Among the halogen-substituted cycloalkyl groups, the preferred are monohalogenocyclopropyl groups and dihalogenocyclopropyl group, and the most preferred is monofluorocyclopropyl groups.

Substituent R⁵ represents hydrogen atom, phenyl group, acetoxymethyl group, pivaloyloxymethyl group, ethoxycarbonyl group, choline group, dimethylaminoethyl group, 5-indanyl group, phthalidinyl group, 5-alkyl-2-oxo-1,3-dioxol-4-ylmethyl group, 3-acetoxy-2-oxobutyl group, an alkyl group containing 1 to 6 carbon atoms, an alkoxymethyl group containing 2 to 7 carbon atoms, or a phenylalkyl group comprising an alkylene group containing 1 to 6 carbon atoms and phenyl group.

When the compound of the present invention (1) is used for an antibacterial purpose, the compound is preferably a carboxylic acid compound wherein the substituent R⁵ is hydrogen atom.

On the other hand, quinolone carboxylic acid derivatives produced by esterifying a carboxylic acid are useful as a synthetic intermediate or a prodrug. The esters which are useful as a synthetic intermediate include alkyl esters, benzyl esters, alkoxyalkyl esters, phenylalkyl esters, and phenyl esters. The esters which are useful as a prodrug include those which are easily cleaved in a living body to produce a free carboxylic acid such as acetoxymethyl ester, pivaloyloxymethyl ester, ethoxycarbonyl ester, choline ester, dimethylaminoethyl ester, 5-indanyl ester, phthalidyl ester, 5-alkyl-2-oxo-1,3-dioxol-4-ylmethyl ester, and 3-acetoxy-2-oxobutyl ester.

Substituents X¹ and X² represent independently hydrogen atom or a halogen atom. Particularly preferable halogen atom is fluorine atom.
Preferable combinations of X¹ and X² include the combination wherein both X¹ and X² are hydrogen atom, and the combination wherein one is hydrogen atom and the other is fluorine atom.

Substituent X represents hydrogen atom or a halogen atom. Particularly preferable halogen atom is fluorine atom.

The compound (1) of the present invention has 4 optical isomers since the 3-amino-4-fluorine substituted methyl pyrrolidine-1-yl group (formula (3)):

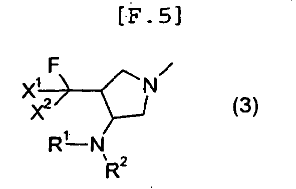

which is the substituent at position 7 has asymmetric carbon atoms at position 3 and position 4. Among such isomers, the preferred are 3,4-cis form, the more preferred are those having (3S,4S) or (3S,4R) configuration, and the most preferred is the one having (3S,4S) configuration (formula (3-1)) :

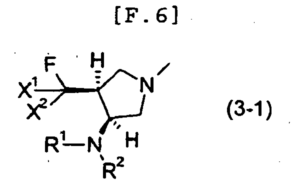

In formula (3) and formula (3-1), R¹, R², X¹ and X² are as defined above.

When R⁴ is a halogen-substituted cycloalkyl group in the compound of the present invention (1), a preferable stereochemical environment is such that the halogen atom and the quinolone carboxylic acid skeleton are in 1,2-cis configuration in relation to the cycloalkane ring. The term "cis configuration" means that the halogen atom and the quinolone carboxylic acid skeleton are in cis configuration in relation to the cycloalkane ring. Of the cis configurations (1R,2S) and (1S,2R), the preferred is (1R,2S) configuration.

When the compound of the present invention represented by the formula (1) is the one having diastereomers, and the compound of the present invention is administered to an animal including human, the compound administered is preferably the one comprising a single diastereomer. The "compound comprising a single diastereomer" includes not only the compound free from the other diastereomer, but also the compound containing the other diastereomer to the extent not affecting the physical constant or activity. In addition, when the compound of the present invention is administered, the compound administered preferably comprises stereochemically single compound. The "compound comprising stereochemically single compound" includes not only the compound comprising only one optically active substance but also the compound containing another optically active substance to the extent not affecting the physical constant or activity when optical isomers are present. The compound of the present invention (1) is most preferably the one having the substituent of position 7 wherein the position 3 and the position 4 are in (3S,4S) configuration, and the halogenocycloalkyl group R⁴ which is in (1R,2S) configuration.

The compound of the present invention (1) may be used as a free substance, but also as an acid addition salt or a salt of the carboxyl group. Exemplary acid addition salts include salts of an inorganic acid such as hydrochloride salt, sulfate salt, nitrate salt, hydrobromate salt, hydroiodate salt, and phosphate salt; sulfonate salts such as methanesulfonate salt, benzenesulfonate salt, and p-toluenesulfonate salt; and salts of an organic salt such as salts of a carboxylic acid such as acetate salt, citrate salt, maleate salt, fumarate salt, and lactate salt. Exemplary salts of carboxyl group include alkaline metal salt such as lithium salt, sodium salt, and potassium salt; alkaline earth metal salts such as magnesium salt and calcium salt; ammonium salts such as triethylamine salt, N-methyl glucamine salt, and tris-(hydroxymethyl)aminomethane salt. The free substance, the acid addition salt, or the salt of carboxyl group may be also present as a hydrate.

Examples of the compound of the present invention (1) include 7-[(3S,4S)-3-amino-4-fluoromethyl-1-pyrrolidinyl]-1-cyclopropyl-6-fluoro-1,4-dihydro-8-methyl-4-oxoquinoline-3-carboxylic acid, or a salt or a hydrate thereof (Compound No. 1); 7-[(3S,4S)-3-amino-4-fluoromethyl-1-pyrrolidinyl]-1-cyclopropyl-1,4-dihydro-8-methyl-4-oxoquinoline-3-carboxylic acid, or a salt or a hydrate thereof (Compound No. 2); 7-[(3S,4S)-3-amino-4-fluoromethyl-1-pyrrolidinyl]-6-fluoro-1-[(2S,1R)-2-fluorocyclopropyl]-1,4-dihydro-8-methyl-4-oxoquinoline-3-carboxylic acid, or a salt or a hydrate thereof (Compound No. 3); 7-[(3S,4S)-3-fluoromethyl-4-methylaminopyrrolidine-1-yl]-6-fluoro-1-[(2S,1R)-2-fluorocyclopropyl]-1,4-dihydro-8-methyl-4-oxoquinoline-3-carboxylic acid, or a salt or a hydrate thereof (Compound No. 4); 1-cyclopropyl-6-fluoro-7-[(3S,4S)-3-fluoromethyl-4-methylaminopyrrolidine-1-yl]-8-methyl-1,4-dihydro-4-oxoquinoline-3-carboxylic acid, or a salt or a hydrate thereof (Compound No. 5); 7-[(3S,4S)-3-amino-4-difluoromethylpyrrolidine-1-yl]-6-fluoro-1-[(1R,2S)-2-fluorocyclopropyl]-8-methyl-1,4-dihydro-4-oxoquinoline-3-carboxylic acid, its salt or a hydrate thereof (Compound No. 6).
Among these, the most preferred are 7-[(3S,4S)-3-amino-4-fluoromethyl-1-pyrrolidinyl]-6-fluoro-1-[(2S,1R)-2-fluorocyclopropyl]-1,4-dihydro-8-methyl-4-oxoquinoline-3-carboxylic acid, or a salt or a hydrate thereof (Compound No. 3); and 7-[(3S,4S)-3-fluoromethyl-4-methylaminopyrrolidine-1-yl]-6-fluoro-1-[(2S,1R)-2-fluorocyclopropyl]-1,4-dihydro-8-methyl-4-oxoquinoline-3-carboxylic acid, its salt or a hydrate thereof (Compound No. 4).

In producing the compound of the present invention (1), the substituent at position 7 may be formed by reacting the following intermediate compound (formula (A)) with an adequate starting compound.

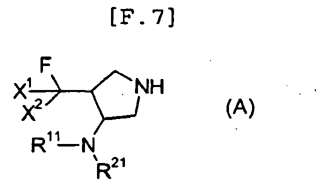

The asymmetry at position 3 and position 4 of this intermediate compound is as described above. Therefore, the compound used in the reaction is preferably the one having (3S,4S) configuration or (3S,4R) configuration, and in particular, the one having (3S,4S) configuration (formula (A-1)).

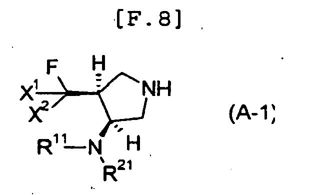

In the compound of formula (A) and formula (A-1), X¹ and X² are as defined above. On the other hand, R¹¹ and R²¹ are the substituents as defined above for the R¹ and R² to which a protective group for the amino group (nitrogen atom) has been added. The protective group of the amino group is not particularly limited as long as protection and deprotection can be readily accomplished with no influence on the reactions of the subsequent steps or the protective group itself does not undergo any reaction. Such protective group of the amino group may be any protective commonly used in the art selected from optionally substituted alkoxycarbonyl groups, optionally substituted aralkyloxycarbonyl groups, optionally substituted acyl groups, optionally substituted aralkyl groups, and substituted silyl groups. More specifically, exemplary optionally substituted alkoxycarbonyl groups include methoxycarbonyl group, ethoxycarbonyl group, tertiary butoxycarbonyl group, and 2,2,2-trichloroethoxycarbonyl group; and exemplary optionally substituted acyl groups include acetyl group, methoxyacetyl group, trifluoroacetyl group, chloroacetyl group, pivaloyl group, formyl group, and benzoyl group; exemplary optionally substituted aralkyloxycarbonyl groups include benzyloxycarbonyl group, p-methoxybenzyloxycarbonyl group, and paranitrobenzyloxycarbonyl group; and exemplary substituted silyl groups include trimethylsilyl group, isopropyldimethylsilyl group, tertiary butyldimethylsilyl group, tribenzylsilyl group, and tertiary butyldimethylsilyl group. Among these, the preferred protective groups used for such intermediate compound are optionally substituted alkoxycarbonyl groups, optionally substituted aralkyloxycarbonyl groups, and optionally substituted acyl group, and the more preferred are methoxycarbonyl group, ethoxycarbonyl group, tertiary butoxycarbonyl group, benzyloxycarbonyl group, acetyl group, and trifluoroacetyl group, and the most preferred is tertiary butoxycarbonyl group.
The nitrogen atom at position 1 of the pyrrolidine ring may also be produced as a compound wherein the nitrogen atom (amino group) has been protected by a protective group. The protective group used in such compound for the protection of the position 1 may also be selected from those described above.
As described above, the intermediate compound may have 3 protective groups of the amino group at most. Such protective groups may be selected as desired by the selection criteria commonly known in the art.

Exemplary preferable methods for producing the compound of the present invention represented by the formula (1) are the methods as described below. Next, the production method is described in detail by using the compound of Example 3 (Compound No. 3) as an example.

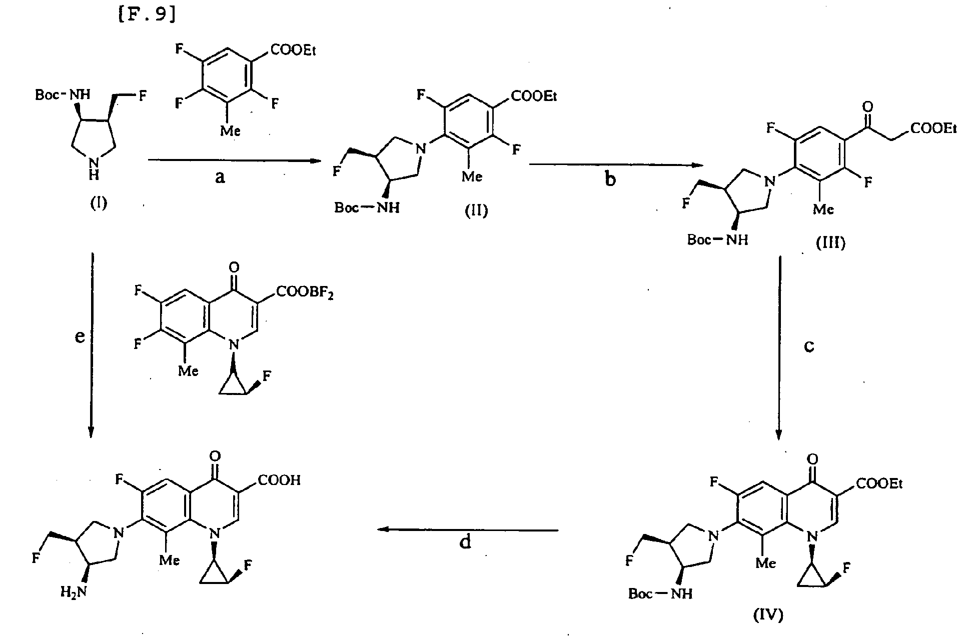

### Compound No. 3

The compound of the present invention can be produced by two methods, namely, by a method wherein 7-halogeno-1,4-dihydro-4-oxoquinoline-3-carboxylic acid derivative is reacted with a pyrrolidine compound for introducing the pyrrolidine substituent, or a method wherein the pyrrolidine compound is reacted with a 4-halogeno benzoic acid derivative and subsequently closing the quinoline ring.
The latter method is first described.

### [Step a] Reaction of pyrrolidine compound with a benzoic acid derivative: Compound (II)

The benzoic acid derivative used in this reaction is preferably a 4-halogeno benzoic acid derivative, and more preferably, a 2,4-dihalogenobenzoic acid derivative. The substituent of the benzoic acid at a position other than such position is also acceptable as long as it corresponds to the substituent of the quinolone compound to be produced. For example, a 8-methyl quinolone derivative may be produced by using a 2,4-dihalogeno-3-methyl benzoic acid derivative. The halogen at position 2 and position 4 may be fluorine atom or chlorine atom, and more preferably, fluorine atom. The substituent at position 4 and position 2, however, is not limited to such a halogen atom as long as it has the function of leaving group.

The carboxy group moiety of the benzoic acid may be either free carboxy group (-COOH) or an ester group (-COOR). Among these, the preferred is an ester group. Exemplary ester groups include alkyl esters, aryl esters, aralkyl esters, phenyl esters (wherein the phenyl group is optionally substituted), and benzyl esters. Among these, use of an alkyl ester is convenient, and the more preferred are methyl ester, ethyl ester, propyl ester, and the like.

The reaction of the pyrrolidine compound (I) with the a benzoic acid derivative is preferably conducted in the presence of a base, and the base used in this reaction is not particularly limited as long as it does not inhibit the reaction. Exemplary bases include organic bases such as trialkylamines (trimethylamine, triethylamine, etc.) and heterocyclic compounds (4-(dimethylamino)pyridine, N-methylmorpholine, 1,8-diazabicyclo[5.4.0]-7-undecene (DBU), etc.); and inorganic salts such as ammonia, ammonia salts, alkaline metal carbonate salts, alkaline earth metal carbonate salts (potassium carbonate, sodium carbonate, etc.), and alkaline metal hydroxides (sodium hydroxide, potassium hydroxide, etc.). Among these, the preferred are organic bases such as a tertiary amine, and in particular triethylamine, and a heterocyclic compound such as 1,8-diazabicyclo [5.4.0]-7-undecene (DBU).
The base is preferably used at an amount of 1 equivalent or more.

Furthermore, HF is produced in this step with the progress of the reaction, and this HF is estimated to cause problems such as leaving of the necessary protective group, formation of a salt with the amine compound to inhibit the reaction with the benzoic acid compound, corrosion of the metal reaction container, and generation of pollution problems. Accordingly, this step is preferably conducted in the presence of a base to prevent such problems. When this step is conducted by using an acid addition product of pyrrolidine compound, a base is also required for the purpose of producing the free base of this salt.

The reaction of the benzoic acid derivative with the pyrrolidine compound is preferably conducted in the presence of a solvent, and the solvent that can be used in this reaction is not particularly limited as long as it does not inhibit the reaction. Exemplary solvents include N-alkylamides such as N,N-dimethylacetamide and N-methylpyrrolidone; aprotic polar solvent such as N,N-dimethylformamide, dimethylsulfoxide, and sulfolane; and acetonitrile; and the preferred are acetonitrile, and N,N-dimethylacetamide (a N-alkylamide).

An adequate reaction temperature in the range between freezing point to the boiling point may be selected. However, the preferable reaction temperature is in the range of room temperature to the boiling point of the reaction solution. The reaction time which is the time from the start to the confirmation of the disappearance of the starting material is generally in the range of 1 hour to 100 hours, and preferably 10 hours to 30 hours.

### [Step b] Reaction of Compound (II) with a half ester of malonic acid: Compound (III)

Next, the benzoic acid derivative (II) having the pyrrolidine substituent introduced is converted into benzoylacetate ester compound (III) . In this step, the benzoate ester may be first hydrolyzed to produce free benzoic acid, and then reacted with the half ester of malonic acid.

The hydrolysis may be conducted under the conditions commonly used in the art for the hydrolysis of an ester, and the conditions may be selected by considering the nature of the protective groups and the substituents at other sites in the compound. In addition to the hydrolysis, this reaction may also be conducted by hydrogenolysis depending on the type of the ester. The hydrolysis is typically conducted under alkaline hydrolytic conditions, and for the handling convenience, an aqueous solution in an alkaline metal hydroxide is preferably reacted at room temperature in a solvent which does not inhibit the reaction and which is miscible with water. This reaction usually proceeds under mild conditions, and at room temperature, the reaction is typically completed in several hours.

The benzoic acid derivative may be separated by extracting under acidic conditions after removing the solvent, and further purified by a chromatographic process, recrystallization, or the like. However, the product is usually usable in the subsequent step (the reaction with the half ester of malonic acid) with no further purification.
Conversion into benzoyl acetic acid compound by the reaction with the malonic acid half ester may be carried out as described below. The malonic acid half ester used may a commercially available product or the one prepared from a diester, and preferably, an alkyl ester in view of the convenience of the preparation. The ester used may be adequately selected by considering the situation of the protective groups and the substituents at other sites in the compound. The malonic acid half ester may be first reacted with a base for conversion into a salt, and then mixed with the benzoic acid derivative that had been obtained to thereby carry out the reaction.

The base used in preparing the malonate salt is preferably a metal alkoxide in view of the handling convenience, and the most preferred are magnesium compounds such as magnesium ethoxide and magnesium chloride. Sodium alkoxide compounds which are often used in the art may also be used.

This reaction can be conducted by using a solvent which does not inhibit the reaction, and exemplary solvents which can be used include anhydrous aprotic solvents such as aromatic hydrocarbons such as benzene, toluene, and xylene; ethers such as dioxane, tetrahydrofuran, and diethyl ether; and dimethyl formamide (DMF) and dimethyl sulfoxide (DMSO). An alcohol corresponding to the alcohol constituting the half ester may also be used.
The reaction between the base and the half ester proceeds quickly, and at room temperature, the reaction is typically completed in several hours.
The reaction of the half ester salt and the benzoic acid derivative may be conducted by activating the benzoic acid derivative, and mixing the activated benzoic acid derivative with the half ester salt.

The activation of the benzoic acid derivative may be accomplished by acid chloride method using thionyl chloride, oxalyl dichloride, phosphorus oxychloride, or the like; a method using a coupling agent such as N,N'-dicyclohexyl carbodiimide (DCC) or 1,1-carbonyldiimidazole (CDI); a method using an azide; a method using mixed acid anhydrides; or a method using an active ester. The method used may be adequately selected from those mentioned above depending on the type and the nature of the substituents and the protective groups in the compound used in the reaction based on the common knowledge of the art.

The reaction with the half ester salt can be conducted by using a solvent which does not inhibit the reaction, and exemplary solvents which can be used include anhydrous aprotic solvents such as aromatic hydrocarbons such as benzene, toluene, and xylene; ethers such as dioxane, tetrahydrofuran, and diethyl ether; and dimethyl formamide (DMF), and dimethyl sulfoxide (DMSO).

The reaction may be carried out at a temperature in the range of ice cold temperature to 200°C, and preferably, at ice cold temperature to 100°C. The mixing of the half ester salt and the activated benzoic acid derivative is preferably conducted in an ice bath. After the mixing,
the reaction may be promoted at a temperature in the range of room temperature to 200°C, preferably, at room temperature to 100°C, and more preferably at room temperature.

### [Step c] Ring closing of Compound (III): Compound (IV)

The benzoylacetate ester compound (III) is first reacted with a N,N-dialkylformaldehyde dialkylacetal, and then, with fluorocyclopropylamine. The ring is then cyclized to produce 1,4-dihydro-4-oxoquinoline-3-carboxylate compound (IV).
The N,N-dialkylformaldehyde dialkylacetal compound used is preferably the one wherein the alkyl groups are independently a lower alkyl group containing 1 to 6 carbon atoms in view of the handling convenience. Exemplary such compound is N,N-dimethylformaldehyde dimethylacetal. The reaction between the N,N-dialkylformaldehyde dialkylacetal compound and the benzoylacetate derivative may be conducted in an adequate solvent.

The solvent which may be used include ethers such as diethyl ether, dioxane, tetrahydrofuran, monoglyme, and diglyme; aromatic hydrocarbons such as benzene, toluene, and xylene; aliphatic hydrocarbons such as n-hexane, heptane, and cyclohexane; and aprotic polar solvents such as DMF, DMSO, and HMPA. The solvent used may also be an anhydrous lower alkane acid such as acetic anhydride.

The reaction is generally conducted at a temperature of 0°C to 200°C, and more preferably, at 0°C to 150°C. The reaction is typically completed in about 0.5 to 10 hours.

The N,N-dialkylformaldehyde dialkylacetal may be used at equimolar amount to significant excess, and preferably at equimolar amount to 2 fold molar excess of the benzoylacetate compound.

The subsequent reaction with the fluorocyclopropylamine may be conducted by reacting the reactants in an adequate solvent.
The solvent used in this step is not particularly limited as long as it does not inhibit the reaction, and exemplary solvents include alcohols such as methanol, ethanol, and propanol; ethers such as diethyl ether, dioxane, tetrahydrofuran, monoglyme, and diglyme; aromatic hydrocarbons such as benzene, toluene, and xylene; aliphatic hydrocarbons such as n-hexane, heptane, cyclohexane, and ligloin; halogenated hydrocarbons such as chloroform, methylene chloride, and carbon tetrachloride; and aprotic polar solvents such as DMF, DMSO, and HMPA. The reaction is generally conducted at a temperature of 0°C to 150°C, and more preferably, at room temperature to 100°C. The reaction is typically completed in about 0.5 to 15 hours.
The amine compound may be used at least at equimolar amount, and preferably at equimolar amount to 2 fold molar excess of the quinolone compound.

If desired, a basic compound may be added to the reaction system. Exemplary basic compounds which may be used include inorganic bases such as metal sodium, metal potassium, metal magnesium, sodium hydride, sodium amide, sodium hydroxide, potassium hydroxide, sodium carbonate, potassium carbonate, and sodium hydrogencarbonate; metal alcoholates such as sodium methylate and sodium ethylate; and organic bases such as heterocyclic compounds (pyridine, piperidine, quinoline, N-methyl morpholine, etc.), trialkylamines (triethylamine, methyl diisopropylamine, etc.), and aryl amines (N,N-dimethylaniline). When the fluorocyclopropylamine is used in the form of a salt, a required additional amount of the base as mentioned above may be added for conversion of the amine salt into the free amine.

The cyclization into the quinolone compound may be conducted in an adequate solvent and in the presence of a basic compound.
The solvent used in this step is not particularly limited as long as it does not inhibit the reaction, and exemplary solvents include ethers such as diethyl ether, dioxane, tetrahydrofuran, monoglyme, and diglyme; aliphatic hydrocarbons such as n-hexane, heptane, and ligroin; halogenated hydrocarbons such as chloroform, methylene chloride, and carbon tetrachloride; and aprotic polar solvents such as DMF, DMSO, and HMPA.

Exemplary basic compounds used include inorganic bases such as metal sodium, metal potassium, sodium hydride, sodium amide, sodium hydroxide, and potassium hydroxide; metal alcoholates such as sodium methylate and sodium ethylate; and organic bases such as 1,8-diazabicyclo[5,4,0]undecene-7 (DBU), N-benzyl trimethylammonium hydroxide, and tetrabutylammonium hydroxide.

The reaction is generally conducted at a temperature of 0°C to 150°C, and more preferably, at room temperature to 120°C. The reaction is typically completed in about 0.5 to 5 hours.

The basic compound is typically used at least at equimolar amount, and preferably at equimolar amount to 2 fold molar excess of the starting compound.

[Step d] Hydrolysis of Compound (IV): Compound No. 3 Hydrolysis of the 1,4-dihydro-4-oxoquinoline-3-carboxylate ester compound (IV) into the carboxylic acid compound can be accomplished under the reaction conditions commonly used in the art for the hydrolysis. More specifically, the reaction may be conducted in the presence of a basic compound such as sodium hydroxide, potassium hydroxide, barium hydroxide, or potassium carbonate; a mineral acid such as sulfuric acid, hydrochloric acid, or nitric acid; or an organic acid such as acetic acid, an alkyl sulfonic acid, or an aromatic sulfonic acid; in the presence of water; an alcohol such as methanol, ethanol, or isopropanol; a ketone such as acetone, methyl ethyl ketone; an ether such as dioxane or ethylene glycol; acetic acid; or a mixture thereof.

The reaction typically proceeds at approximately room temperature to 200°C, and more preferably at approximately room temperature to 150°C. The reaction is typically completed in about 0.5 to 30 hours.

Next, the former method is described.
The method as described above (the latter method) wherein ring closing of the quinoline is conducted after introducing the pyrrolidine substituent moiety is conducted on the benzoic acid derivative before introducing the pyrrolidine substituent moiety to thereby construct the quinolone skeleton and obtain 7-halogeno-1,4-dihydro-4-oxoquinoline-3-carboxylic acid, and the pyrrolidine substituent is thereafter introduced in the 7-halogeno-1,4-dihydro-4-oxoquinoline-3-carboxylic acid. Alternatively, the benzoic acid derivative may be reacted with a malonic acid compound, and the resulting benzoylacetate compound may be reacted with the pyrrolidine compound.

### [Step e] Introduction of pyrrolidine substituent: Compound No. 3

In this step, 7-halogeno-1,4-dihydro-4-oxoquinoline-3-carboxylic acid or a compound (boron chelate compound) wherein the carboxyl group moiety of the 7-halogeno-1,4-dihydro-4-oxoquinoline-3-carboxylic acid has been converted to di-substituted boron oxycarbonyl structure is reacted with the pyrrolidine compound to introduce the substituent.

In the reaction of the 7-halogeno-1,4-dihydro-4-oxoquinoline-3-carboxylic acid or its boron chelate compound with the pyrrolidine compound, the reactants may be used at any ratio selected from a wide range. However, the pyrrolidine compound is typically used at least at approximately equimolar amount, and preferably, at equimolar amount to 5 fold molar excess of the 7-halogeno-1,4-dihydro-4-oxoquinoline-3-carboxylic acid or its boron chelate compound.
This reaction may be carried out in a solvent which does not inhibit the reaction. Exemplary such solvents include water; alcohols such as methanol, ethanol, isopropanol, butanol, amyl alcohol, and isoamyl alcohol; aromatic hydrocarbons such as benzene, toluene, and xylene; ethers such as tetrahydrofuran, dioxane, and diglyme; aprotic (polar) solvents such as dimethylacetamide, DMF, DMSO, HMPA, and N-methylpyrrolidone; and mixtures thereof. Among these, the preferred are DMF, DMSO, HMPA, and N-methylpyrrolidone. The reaction may also be conducted in the presence of a deoxidizer such as an inorganic carbonate salt such as sodium carbonate, potassium carbonate, sodium hydrogencarbonate, and potassium hydrogencarbonate; or an organic base such as pyridine, quinoline, or triethylamine. An alkaline metal halide such as potassium fluoride may also be added to the reaction system.

This reaction is typically conducted at a pressure of 1 to 20 atm., and preferably at 1 to 10 atm. and at a temperature of room temperature to about 250°C, and preferably at room temperature to 200°C. The reaction is typically completed in about 0.5 to 30 hours.

When the carboxylic acid moiety has a boron-containing structure, the compound may be treated with an acidic or basic compound to decompose the chelate and derive the corresponding carboxylic acid compound. The acids which may be used in such step include mineral acids such as hydrochloric acid and sulfuric acid, and organic acids such as acetic acid and p-toluenesulfonic acid. The basic compounds which may be used include inorganic bases such as sodium hydroxide, potassium hydroxide, sodium hydrogencarbonate, potassium hydrogencarbonate, and potassium carbonate and organic bases such as triethylamine. The reaction proceeds at a temperature of approximately 0 to 150°C, and preferably at approximately 0 to 100°C. The acid or the basic compound is typically used at least at equimolar amount, and preferably at equimolar amount to 10 fold molar excess of the starting compound.

Examples of the compound (boron chelate compound) having the di-substituted boron oxycarbonyl structure include dihalogenoboron and dialkanoyl oxyboron. Preferable examples of the dihalogeno compound include difluoroboron, and preferable examples of the dialkanoyl oxyboron include diacetoxy boron. Among these, the preferred is difluoroboron in view of the handling convenience.

The difluoroboron oxy compound (difluoroboron chelate compound) may be produced by reacting the carboxylic acid compound with an ether complex of boron trifluoride, for example, diethyl ether complex or tetrahydrofuran complex, or alternatively, by treating the carboxylic acid compound with tetrafluoroboric acid.

This boron chelate moiety should be cleaved at a certain stage to regenerate the carboxy group, and the cleavage can be accomplished by hydrolysis under basic or acidic conditions. This step can be accomplished by any of the known methods.

Since the compound of the present invention (1) has strong antibacterial activity and high safety with reduced side effects such as heart toxicity, it can be used as a drug for human, animals, and fish, or as a preservative of agricultural chemicals and foods. The dose of the compound of the present invention (1) when it is administered as a drug may vary according to the age, sex, and symptoms of the patients. However, the dose is typically 50 mg to 1 g, and more preferably 100 mg to 500 mg per day per adult. When the compound of the invention is administered to an animal, the dose is typically 1 mg to 200 mg, and more preferably 5 mg to 100 mg per day per kg weight of the animal although the dose may vary according to the size of the animal to be treated, type of the pathogenic microorganism, and seriousness of the condition. Such daily dose may be administered in a single dose or in 2 to 4 divided doses. If necessary, a dose exceeding such daily dose may be administered.

The compound of the present invention (1) has excellent antibacterial activity for a broad range of microorganisms causing various infections, and therefore, the present compound is capable of treating, preventing, or ameliorating the diseases caused by such pathogenic microorganisms. The compound of the present invention (1) is effective for bacteria and the bacteria-like microorganisms including Staphylococcus, Streptococcus pyogenes, hemolytic streptococcus, enterococcus, pneumococcus, Peptostreptococcus, Neisseria gonorrhoeae, Escherichia coli, Citrobacter, Shigella, Klebsiella pneumoniae, Enterobacter, Serratia, Proteus, Pseudomonas aeruginosa, Haemophilus influenzae, Acinetobacter, Campylobacter, and Chlamydia trachomatis.

The diseases caused by such pathogenic microorganisms include superficial secondary infections such as folliculitis, furuncle, carbuncle, erysipelas, cellulitis, lymphangitis, whitlow, subepidermal abscess, hidradenitis, acne conglobata, infectious atheroma, perianal abscess, mastitis, and injury, burn and operative wounds; secondary infections of laryngopharyngitis, acute bronchitis, tonsillitis, chromic bronchitis, bronchiectasis, diffuse panbronchiolitis, and chronic respiratory diseases; pneumonia, pyelonephritis, cystitis, prostatitis, epididymitis, gonorrheal urethritis, nongonococcal urethritis, cholecystitis, cholangitis, shigellosis, enteritis, adnexitis, intrautarine infection, bartholinitis, blepharitis, hordeolum, dacryocystitis, meibomianitis, corneal ulcer, middle otitis, sinusitis, periodontal inflammations, pericoronitis, jaw inflammation, peritonitis, endocarditis, sepsis, meningitis, and skin infections.

The compound of the present invention (1) is also effective for acid fast bacteria such as M. tuberculosis complex (Mycobacterium tuberculosis, M. bovis, and M. africans) and atypical mycobacteria (M. kansasii, M. marianum, M. scrofulaceum, M.avium, M. intracellulare, M. xenopi, M. fortuitum, and M. chelonae). The mycobacterial infections caused by such pathogenic microorganisms are divided into three categories of tuberculosis, atypical mycobacteriosis, and leprosy. Mycobacterial infections affect not only the lung but also thoracic cavity, trachea and bronchus, lymph nodes, by systemic dissemination, joints and bones, meninges and brain, digestive organs (intestine and liver), skin, mammary gland, eyes, auris media and throat, urinary tract, male genitalia, and female genitalia. The main organ affected by the atypical mycobacteriosis (non-tuberculous mycobacteriosis) is lung. The atypical mycobacteriosis, however, also affects by topical lymphadenitis, skin soft tissues, bones and joints, and by systemic dissemination.

The compound of the present invention is also effective for various microorganisms causing animal infections such as Escherichia, Salmonella, Pasteurella, Haemophilus, Bordetella, Staphylococcus, and mycoplasma. Exemplary diseases include, colibacillosis, pullorum disease, avian paratyphoid, fowl cholera, infectious diarrhea, staphylococcosis, mycoplasma infection, and the like for fowls; colibacillosis., salmonellosis, pasteurellosis, hemophilosis, atrophic rhinitis, exudative epidermitis, mycoplasma infection and the like for pigs; colibacillosis, salmonellosis, hemorrhagic septicemia, mycoplasma infection, pleuropneumonia, and mastitis for cows; Escherichia coli sepsis, salmonnella infection, hemorrhagic septicemia, pyometra, cystitis, and the like for dogs; and exudative pleurisy, cystitis, chronic rhinitis, hemophilosis, kitten's diarrhea, mycoplasma infection, and the like for cats.

The drug of the present invention contains the compound of the present invention (1), or a salt or a hydrate thereof as its effective component, and the nonlimiting dosage form may be adequately selected. Exemplary dosage forms include oral solid and liquid preparations such as tablet, powder, granules, capsule, solution, syrup, elixir, and oil-base and water-base suspension; non-oral preparations such as injection and suppository; external preparation, instillation, and patch. The dosage form may be prepared by any of common method used for producing various preparations by blending with a pharmaceutically acceptable carrier.
In the case of an injection, the preparation may contain a stabilizer, an antiseptic, a solubilizer, and the like and the preparation optionally supplemented with such additives may be filled in a container, and then freeze dried to produce a solid preparation to be hydrated immediately before use. The container may be filled either with a single dose or multiple doses.
In the case of an external preparation, the preparation may be, for example, a solution, a suspension, an emulsion, an ointment, a gel, a cream, a lotion, or a spray.
In the case of a solid preparation, the preparation may contain a pharmaceutically acceptable carrier with the compound (1), and exemplary carries include fillers, expanders, binders, disintegrants, solubilizers, wetting agents, and lubricants. The liquid preparation may be a solution, a suspension, an emulsion, or the like which may contain a suspending agent or emulsifier as an additive.

The compound of the present invention (1) may be administered to animals, for example, by direct oral administration, by adding the compound to the feed for oral administration, by dissolving the compound and adding the solution to the drinking water or the feed for oral administration, or by injection.

Next, exemplary preparations are described.

**Preparation 1 (Capsule)**

| | |
|---|---|
| Compound of Example 1 | 100.0 mg |
| Corn starch | 23.0 mg |
| CMC calcium | 22.5 mg |
| Hydroxymethylcellulose | 3.0 mg |
| Magnesium stearate | 1.5 mg |
| Total | 150.0 mg |

**Preparation 2 (Solution)**

| | |
|---|---|
| Compound of Example 1 | 1 to 10 g |
| Acetic acid or sodium hydroxide | 0.5 to 2 g |
| Ethyl paraoxybenzoate | 0.1 g |
| Purified water | 88.9 to 98.4 g |
| Total | 100.0 g |

**Preparation 3 (Powder for animal feed)**

| | |
|---|---|
| Compound of Example 1 | 1 to 10 g |
| Corn starch | 98.5 to 89.5 g |
| Light anhydrous silicic acid | 0.5 g |
| Total | 100.0 g |

### EXAMPLES

Next, the present invention is described in further detail by referring to Reference Examples and Examples which by no means limit the scope of the present invention.

### [Example 1]

7-[(3S,4S)-3-amino-4-fluoromethyl-1-pyrrolidinyl]-1-cyclopropyl-6-fluoro-1,4-dihydro-8-methyl-4-oxoquinoline-3-carboxylic acid (Compound No. 1)

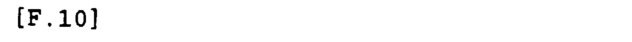

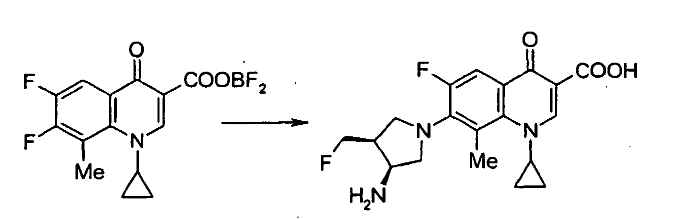

1-cyclopropyl-6,7-difluoro-1,4-dihydro-8-methyl-4-oxoquinoline-3-carboxylic acid - difluoroboron complex (654 mg, 2 mmol) and (3S,4S)-3-amino-4-fluoromethylpyrrolidine dihydrochloride (764 mg, 4 mmol) were dissolved in anhydrous dimethyl sulfoxide (10 ml), and triethylamine (2.23 ml, 16 mmol) was added to the solution. The mixture was stirred at 50°C for 24 hours in nitrogen atmosphere. The solvent was distilled off under reduced pressure, and 90% ethanol (10 ml) and triethylamine (0.5 ml) were added to the residue. After heating under reflux for 3 hours, the solvent was distilled off under reduced pressure. Concentrated hydrochloric acid (6 ml) was added in an ice bath, and the mixture was stirred for 30 minutes, and washed three times with chloroform. The resulting aqueous layer was adjusted to pH 12 by adding saturated aqueous sodium hydroxide solution in an ice bath, and the solution was stirred for 1 hour. Diluted hydrochloric acid was then added to adjust the pH to 7.4, and the solution was stirred for 12 hours. The solution was extracted with chloroform, and dried with anhydrous sodium sulfate. After filtration, the filtrate was concentrated under reduced pressure, and the concentrate was purified by recrystallization from a mixed solvent of diethyl ether and 2-propanol, and drying at reduced pressure at 50°C for 14 hours to thereby obtain 274 mg (0.72 mmol, 36%) of the title compound as white crystals.
¹H-NMR (400 MHz, DMSO-d6) δ: 0.82-0.97 (2H, m), 1.02-1.03 (1H, m), 1.15-1.23 (2H, m), 2.49 (3H, s), 2.59-2.66 (1H, m), 2.75-2.76 (1H, m), 3.18-3.30 (2H, m), 3.46-3.53 (1H, m), 3.60-3.66 (2H, m), 3.82-3.85 (1H, m), 4.28-4.32 (1H, m), 4.52-4.84 (2H, m), 7.69 (1H, d, J = 13.69 Hz), 8.76 (1H, s).
Melting point: 160-165°C (diethyl ether/2-propanol)
Elementary analysis: as C₁₉H₂₁F₂N₃O₃·0.25H₂O
Calculated: C, 59.76%; H, 5.67%; N, 11.00%.
Measured: C, 59.86%; H, 5.63%; N, 10.99%.

### [Example 2]

7-[(3S,4S)-3-amino-4-fluoromethyl-1-pyrrolidinyl]-1-cyclopropyl-1,4-dihydro-8-methyl-4-oxoquinoline-3-carboxylic acid (Compound No. 2)

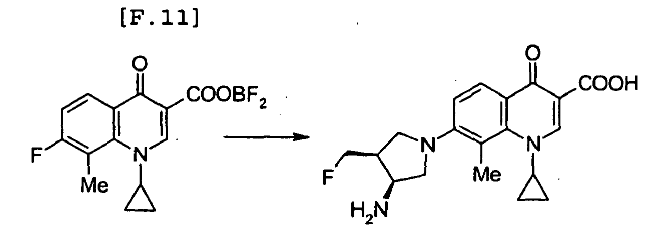

Preparation 3 (Powder for animal feed) 1-cyclopropyl-7-fluoro-1,4-dihydro-8-methyl-4-oxoquinoline-3-carboxylic acid - difluoroboron complex (618 mg, 2 mmol) and (3S,4S)-3-amino-4-fluoromethylpyrrolidine dihydrochloride (764 mg, 4 mmol) were dissolved in anhydrous dimethyl sulfoxide (10 ml), and triethylamine (2.23 ml, 16 mmol) was added to the solution. The mixture was stirred at 50°C for 13 hours in nitrogen atmosphere. The solvent was distilled off under reduced pressure, and 90% ethanol (10 ml) and triethylamine (0.5 ml) were added to the residue. After heating under reflux for 2.5 hours, the solvent was distilled off under reduced pressure. Concentrated hydrochloric acid (6 ml) was added in an ice bath, and the mixture was stirred for 15 minutes, and washed three times with chloroform. The resulting aqueous layer was adjusted to pH 12 by adding saturated aqueous sodium hydroxide solution in an ice bath, and the solution was stirred for 1 hour. Diluted hydrochloric acid was then added to adjust the pH to 7.4, and the solution was stirred for 12 hours. The solution was extracted with chloroform, and dried with anhydrous sodium sulfate. After filtration, the filtrate was concentrated under reduced pressure, and the concentrate was purified by recrystallization from a mixed solvent of diethyl ether and 2-propanol, and drying at reduced pressure at 70°C for 24 hours to thereby obtain 490 mg (1.35 mmol, 67%) of the title compound as yellow crystals.
¹H-NMR (400 MHz, DMSO-d6) δ: 0.82-0.93 (2H, m), 1.02-1.03 (1H, m), 1.18-1.22 (2H, m), 2.49 (3H, s), 2.61-2.66 (1H, m), 3.13-3.15 (1H, m), 3.28-3.40 (2H, m), 3.54-3.59 (1H, m), 3.63-3.66 (1H, m), 3.70-3.74 (1H, m), 4.27 (1H, m), 4.52-4.84 (2H, m), 7.08 (1H, d, J = 9.05 Hz), 7.98 (1H, d, J = 9.05 Hz), 8.73 (1H, s).
Melting point: 203-205°C (diethyl ether/2-propanol)
Elementary analysis: as C₁₉H₂₂FN₃O₃·0.25H₂O
Calculated: C, 62.71%; H, 6.23%; N, 11.55%.
Measured: C, 62.59%; H, 6.16%; N, 11.44%.

### [Example 3]

7-[(3S,4S)-3-amino-4-fluoromethyl-1-pyrrolidinyl]-6-fluoro-1-[(2S,1R)-2-fluorocyclopropyl]-1,4-dihydro-8-methyl-4-oxoquinoline-3-carboxylic acid (Compound No. 3)

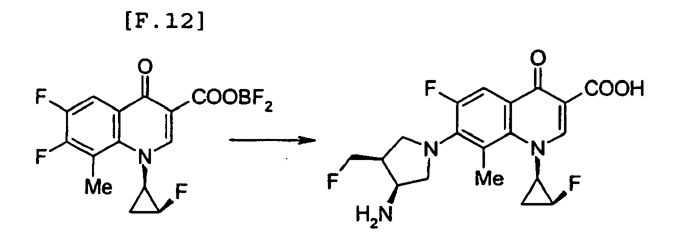

To a solution of 6,7-difluoro-1-[(2S,1R)-2-fluorocyclopropyl] -1,4-dihydro-8-methyl-4-oxoquinoline-3-carboxylic acid - difluoroboron complex (2.23 g, 6.46 mmol) in dimethyl sulfoxide (11 ml) were added 3-(S)-tertiary butoxycarbonylamino-4-(S)-fluoromethylpyrrolidine (1.67 g, 7.65 mmol) and triethylamine (2.16 ml, 15.5 mmol), and the mixture was stirred at 35 to 40°C for 7 days. The reaction solution was concentrated at reduced pressure, and the concentrate was dissolved in a mixed solution of ethanol and water (9 : 1) (150 ml). After adding triethylamine (5 ml), the mixture was heated under reflux for 4 hours. The reaction mixture was concentrated under reduced pressure, and the concentrate was dissolved in ethyl acetate (100 ml x 2), and washed with water (50 ml x 3) and saturated aqueous solution of sodium chloride (50 ml). The organic layer was dried with anhydrous sodium sulfate, and the solvent was distilled off under reduced pressure. The residue was dissolved in concentrated hydrochloric acid (20 ml) in an ice bath, and the aqueous solution was washed with chloroform (50 ml x 3). To the aqueous layer was added 10 mol/l aqueous solution of sodium hydroxide to adjust pH to 12.0, and the basic aqueous solution was adjusted with hydrochloric acid to pH 7.4. The solution was extracted with chloroform (100 ml x 2) and a mixed solution of chloroform and methanol (9 : 1) (100 ml x 5). The organic layer was dried with anhydrous sodium sulfate, and the solvent was distilled off under reduced pressure. The residue was purified by preparative chromatography, and further purified by recrystallization from ethanol, and subsequently, dried under reduced pressure to produce the title compound 175 mg (7%) as pale yellow crystals.
¹H-NMR (400 MHz, 0.1N-NaOD) δ: 1.19-1.31 (1H, m), 1.56-1.66 (1H, m), 2.50 (3H, s), 2.75-2.85 (1H, m), 3.17-3.21 (1H, m), 3.41 (1H, t, J = 8.8 Hz), 3.61-3.72 (2H, m), 3.95-4.11 (2H, m), 4.79-4.88 (3H, m), 7.68 (1H, d, J = 14.2 Hz), 8.46 (1H, s).
IR (ATR)3404, 3336, 3076, 2879, 1707, 1618, 1514, 1468, 1437, 1398, 1363, 1309, 1236cm⁻¹
Melting point: 214-216°C (decomposition)
Elementary analysis: as C₁₉H₂₀F₃N₃O₃·0.25H₂O
Calculated: C, 57.07%; H, 5.17%; F, 14.25%; N, 10.51%
Measured: C, 56.92%; H, 5.07%; F, 14.17%; N, 10.41%

### [Example 4]

7-[(3S,4S)-3-fluoromethyl-4-methylaminopyrrolidine-1-yl]-6-fluoro-1-[(2S,1R)-2-fluorocyclopropyl]-1,4-dihydro-8-methyl-4-oxoquinoline-3-carboxylic acid (Compound No. 4)

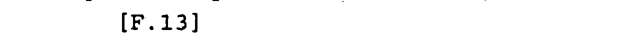

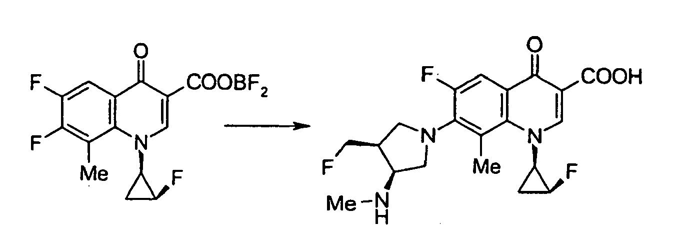

To a solution of (3S,4S)-3-(N-tert-butoxycarbonyl-N-methyl)amino-4-fluoromethylpyrrolidine (8.62 g, 37.1 mmol) in sulfolane (45 ml) were added triethylamine (2.22 ml, 17.4 mmol) and 6,7-difluoro-1-[(1R,2S)-2-fluorocyclopropyl]-8-methyl-1,4-dihydro-4-oxoquinoline-3-carboxylic acid - difluoroboron complex (4.59 g, 13.3 mmol), and the mixture was stirred at 35 to 39°C for 4 days. To the reaction solution were added a mixed solution of ethanol and water (5 : 1) (240 ml) and triethylamine (5 ml), the mixture was heated under reflux 2 hours. The reaction mixture was concentrated under reduced pressure, and the concentrate was dissolved in ethyl acetate (400 ml), and washed with 10% aqueous solution of citric acid (100 ml), water (100 ml x 3), and saturated aqueous solution of sodium chloride (100 ml). The organic layer was dried with anhydrous sodium sulfate, and the solvent was distilled off under reduced pressure. The residue was purified by short silica gel column chromatography (chloroform-methanol; 49 : 1 → 9 : 1), and dissolved in concentrated hydrochloric acid (20 ml) in an ice bath. The solution was stirred at room temperature for 30 minutes, and the reaction solution was washed with chloroform (100 ml x 6). 10 mol/l aqueous solution of sodium hydroxide was added to the aqueous layer in an ice bath to adjust the pH to 12.0, and then, hydrochloric acid was added to adjust the pH to 7.4. The solution was extracted with chloroform (200 ml x 4). The organic layer was dried with anhydrous sodium sulfate, and the solvent was distilled off under reduced pressure. The residue was purified by recrystallization from ethanol (using active carbon), and dried under reduced pressure to produce 1.39 g (26%) of the title compound as pale yellow crystals. Melting point: 173-175°C.
¹H-NMR (400 MHz, 0.1N-NaOD) δ: 1.26-1.38 (1H, m), 1.58-1.69 (1H, m), 2.36 (3H, s), 2.54 (3H, s), 2.82-2.93 (1H, m), 3.41 (1H, q, J = 5.0 Hz), 3.49 (1H, q, J = 5.8 Hz), 3.58 (2H, d, J = 6.9 Hz), 3.79 (1H, ddd, J = 9.6, 6.1, 1.5 Hz), 4.12 (1H, dt, J = 8.6, 5.4 Hz), 4.72-4.80 (2H, m), 5.00 (1H, d, J = 65.0 Hz), 7.70 (1H, d, J = 14.0 Hz), 8.48 (1H, d, J = 2.7 Hz).
Elementary analysis: as C₂₀H₂₂F₃N₃O_{3·}0.25H₂O
Calculated: C, 58.04; H, 5.48; F, 13.77; N, 10.15.
Measured: C, 58.25; H, 5.52; F, 13.76; N, 10.03. IR (ATR): 3329, 2945, 2893, 1726, 1610, 1547, 1502, 1429, 1354, 1315, 1263, 1221 cm⁻¹.

### [Example 5]

1-cyclopropyl-6-fluoro-7-[(3S,4S)-3-fluoromethyl-4-methylaminopyrrolidine-1-yl]-8-methyl-1,4-dihydro-4-oxoquinoline-3-carboxylic acid (Compound No. 5)

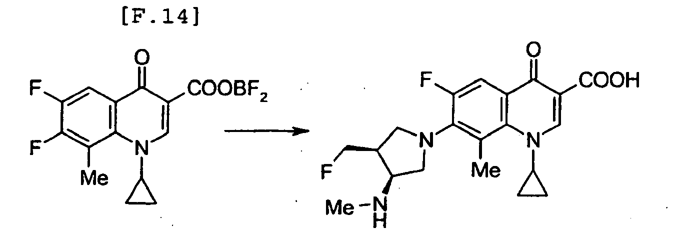

To a solution of (3S,4S)-3-(N-tert-butoxycarbonyl-N-methyl)amino-4-fluoromethylpyrrolidine (651 mg, 2.80 mmol) in sulfolane (3.5 ml) were added triethylamine (293 µl, 2.10 mmol) and 1-cyclopropyl-6,7-difluoro-8-methyl-1,4-dihydro-4-oxoquinoline-3-carboxylic acid - difluoroboron complex (458 mg, 1.40 mmol), and the mixture was stirred at 31 to 35°C for 6 days. Cold water (200 ml) was added to the reaction solution, and the precipitated solid was collected by filtration, and washed with water. To this solid was added a mixed solution of ethanol and water (10 : 1) (165 ml) and triethylamine (3 ml), and the mixture was heated under reflux for 2 hours. The reaction mixture was concentrated under reduced pressure, and the concentrate was dissolved in ethyl acetate (400 ml), and the solution was washed with 10% aqueous solution of citric acid (100 ml), water (100 ml x 2), and saturated aqueous solution of sodium chloride (100 ml). The organic layer was dried with anhydrous sodium sulfate, and the solvent was distilled off under reduced pressure. The residue was dissolved in concentrated hydrochloric acid (5 ml) in an ice bath, and the solution was stirred at room temperature for 30 minutes. The reaction solution was washed with chloroform (100 ml x 3). 10 mol/l aqueous solution of sodium hydroxide was added to the aqueous layer in an ice bath to adjust the pH to 12.0, and hydrochloric acid was then added to adjust the pH to 7.4. The solution was extracted with chloroform (200 ml x 4). The organic layer was dried with anhydrous sodium sulfate, and the solvent was distilled off under reduced pressure. The residue was purified by recrystallization from ethanol, and dried under reduced pressure to produce 161 mg (29%) of the title compound as pale yellow crystals.
Melting point: 156-158°C.
¹H-NMR (400 MHz, 0.1N-NaOD) δ : 0.75-0.88 (2H, m), 1.11-1.22 (2H, m), 2.37 (3H, s), 2.55 (3H, s), 2.79-2.91 (1H, m), 3.47 (3H, dq, J = 21.4, 5.1 Hz), 3.61-3.67 (1H, m), 3.73 (1H, t, J = 8.5 Hz), 4.09-4.15 (1H, m), 4.59-4.77 (2H, m), 7.66 (1H, d, J = 14.0 Hz), 8.57 (1H, s).
Elementary analysis: as C₂₀H₂₃F₂N₃O₃
Calculated: C, 61.37; H, 5.92; F, 9.71; N, 10.74.
Measured: C, 61.18; H, 6.06; F, 9.85; N, 10.68.
IR (ATR): 2889, 1720, 1614, 1545, 1504, 1452, 1429, 1360, 1313, 1259, 1227 cm⁻¹.

### [Example 6]

7-[(3S,4S)-3-amino-4-difluoromethylpyrrolidine-1-yl]-6-fluoro-1-[(1R,2S)-2-fluorocyclopropyl]-8-methyl-1,4-dihydro-4-oxoquinoline-3-carboxylic acid (Compound No. 6)

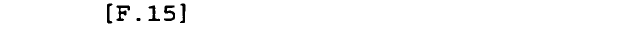

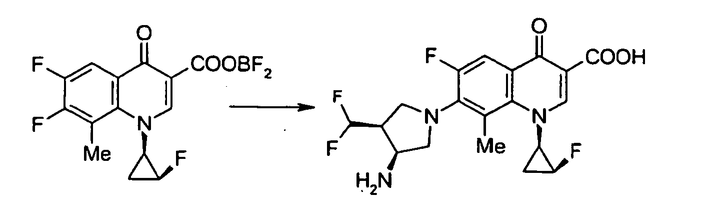

To a solution of (3S,4S)-3-(tert-butoxycarbonyl)amino-4-difluoromethylpyrrolidine (501 mg, 2.12 mmol) in sulfolane (2.5 ml) were added triethylamine (197 µl, 1.41 mmol) and 6,7-difluoro-1-[(1R,2S)-2-fluorocyclopropyl]-8-methyl-1,4-dihydro-4-oxoquinoline-3-carboxylic acid - difluoroboron complex (406 mg, 1.18 mmol), and the mixture was stirred at room temperature for 5 days, and at 35°C for 7 days. Cold water (100 ml) was added to the reaction solution, and the precipitated solid was collected by filtration and washed with water. To this solid were added a mixed solution of ethanol and water (9 : 1) (100 ml) and triethylamine (1 ml), and the mixture was heated under reflux for 30 minutes. The reaction mixture was concentrated under reduced pressure, and the residue was dissolved in ethyl acetate (300 ml), and washed with 10% aqueous solution of citric acid (100 ml), water (100 ml x 3), and saturated aqueous solution of sodium chloride (100 ml). The organic layer was dried with anhydrous sodium sulfate, and the solvent was distilled off under reduced pressure. The residue was dissolved in concentrated hydrochloric acid (5 ml) in an ice bath, and the solution was stirred at room temperature for 30 minutes, and the reaction solution was washed with chloroform (50 ml x 3). 10 mol/l aqueous solution of sodium hydroxide was added to the aqueous layer in an ice bath to adjust the pH to 12.0, and hydrochloric acid was then added to adjust the pH to 7.4. The solution was extracted with chloroform (150 ml x 4). The organic layer was dried with anhydrous sodium sulfate, and the solvent was distilled off under reduced pressure. The residue was purified by preparative silica gel thin layer chromatography (developed in the lower layer of chloroform : methanol : water, 7 : 3 : 1), and further purified by recrystallization from ethanol-diethyl ether, and dried under reduced pressure to produce 85 mg (17%) of the title compound was produced as pale yellow crystals. Melting point: 214-216°C.
¹H-NMR (400 MHz, 0.1N-NaOD) δ: 1.28 (1H, d, J = 27.3 Hz), 1.57-1.68 (1H, m), 2.56 (3H, s), 2.92 (1H, brs), 3.22 (1H, d, J = 10.7 Hz), 3.45 (1H, t, J = 9.0 Hz), 3.81 (1H, brs), 3.90 (1H, t, J = 9.5 Hz), 3.98-4.03 (1H, m), 4.08-4.15 (1H, m), 5.02 (1H, d, J = 66.4 Hz), 6.22 (1H, td, J = 55.7, 6.3 Hz), 7.72 (1H, d, J = 13.9 Hz), 8.47 (1H, d, J = 3.2 Hz).
Elementary analysis: as C₁₉H₁₉F₄N₃O·0.25H₂O
Calculated: C, 54.61; H, 4.70; F, 18.19; N, 10.06.
Measured: C, 54.37; H, 4.51; F, 17.71; N, 10.02.
IR (ATR): 3408, 3336, 3072, 3030, 2947, 2891, 1711, 1618, 1514, 1468, 1439, 1402, 1352, 1306, 1232 cm⁻¹.

### [Reference Example 1]

Ethyl 4-[(3S,4S)-3-tertiary butoxycarbonylamino-4-fluoromethyl-1-pyrrolidinyl]-2,5-difluoro-3-methylbenzoate

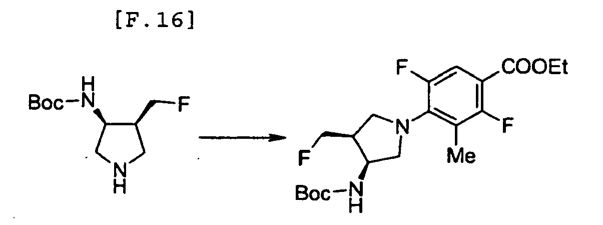

To a solution of ethyl 2,4,5-trifluoro-3-methyl benzoate (1.12 g, 5.14 mmol) in dimethyl sulfoxide (5 ml) were added 3-(S)-tertiary butoxycarbonylamino-4-(S)-fluoromethylpyrrolidine (0.751 g, 3.44 mmol) and 1,8-diazabicyclo[5.4.0]-7-undecene (0.695 ml, 5.14 mmol), and the mixture was stirred at 60 to 65°C for 20 hours. The solution was allowed to cool to room temperature, and the reaction mixture was dissolved in ethyl acetate (50 ml x 2), and the solution was washed with 10% aqueous solution of citric acid (50 ml), water (50 ml x 2), and saturated aqueous solution of sodium chloride (50 ml). The organic layer was dried with anhydrous sodium sulfate, and the solvent was distilled off under reduced pressure. The residue was subjected to silica gel column chromatography, and from the eluate of n-hexane and ethyl acetate (3 : 1) was obtained 725 mg (51%) of the title compound as colorless oily crystals.
¹H-NMR (400 MHz, CDCl₃) δ: 1.38 (3H, t, J = 7.1 Hz), 1.46 (9H, s), 2.22 (3H, d, J = 2.9 Hz), 2.73-2.88 (1H, m), 3.14-3.18 (1H, m), 3.35-3.50 (2H, m), 3.70 (1H, ddd, J = 9.7, 6.0, 1.9 Hz), 4.36 (2H, q, J = 7.1 Hz), 4.47-4.77 (3H, m), 4.92-4.89 (1H, m), 7.44 (1H, dd, J = 12.7, 6.8 Hz).

### [Reference Example 2]

Ethyl 4-[(3S,4S)-3-tertiary butoxycarbonylamino-4-fluoromethyl-1-pyrrolidinyl]-2,5-difluoro-3-methylbenzoylacetate

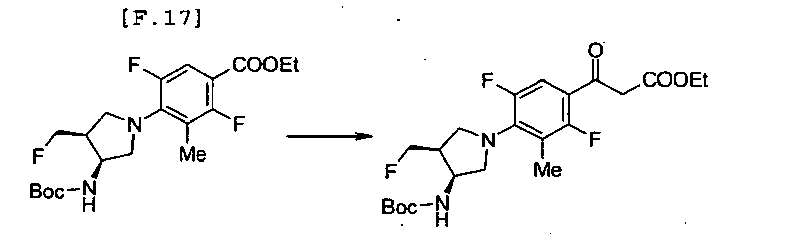

To a solution of ethyl 4-[(3S,4S)-3-tertiary butoxycarbonylamino-4-fluoromethyl-1-pyrrolidinyl]-2,5-difluoro-3-methylbenzoate (720 mg, 1.73 mmol) in ethanol (10 ml) was added 3 mol/l aqueous solution of potassium hydroxide (2.31 ml), and the mixture was stirred at room temperature for 1 hour. To the reaction solution were added 10% aqueous solution of citric acid (10 ml) and water (10 ml) to adjust the pH to 2 to 3, and ethanol was concentrated under reduced pressure. The solution was extracted with chloroform (30 x 2 ml), and dried with anhydrous sodium sulfate. After filtration, the filtrate was concentrated under reduced pressure to produce 4-[(3S,4S)-3-tertiary butoxycarbonylamino-4-fluoromethyl-1-pyrrolidinyl]-2,5-difluoro-3-methyl benzoic acid (718 mg, 1.73 mmol) as a yellow oily product.
Monoethyl malonate (459 mg, 3.48 mmol) was dissolved in anhydrous tetrahydrofuran (5 ml), and magnesium ethoxide (370 mg, 3.23 mmol) was added in an ice bath. The mixture was stirred at room temperature for 2 hours, and the reaction solution was concentrated under reduced pressure to produce magnesium salt of monoethyl malonate. Next, 4-[(3S,4S)-3-tertiary butoxycarbonylamino-4-fluoromethyl-1-pyrrolidinyl]-2,5-difluoro-3-methylbenzoic acid (718 mg, 1.73 mmol) was dissolved in tetrahydrofuran (10 ml), and 1,1-carbonyl diimidazole (365 mg, 2.25 mmol) was added in an ice bath. The mixture was stirred at room temperature for 2 hours, and to this mixture was added dropwise a solution of the magnesium salt of monoethyl malonate prepared as described above in anhydrous tetrahydrofuran (5 ml) in an ice bath. After the completion of the dropwise addition, the solution was allowed gradually to resume room temperature, and the solution was stirred for 16 hours. To this reaction solution was added toluene (10 ml) and 10% aqueous solution of citric acid (10 ml) in an ice bath to acidify the reaction solution (to pH 2 to 3), and the solution was stirred at room temperature for 1 hour. The organic layer was collected and washed with saturated aqueous solution of sodium bicarbonate (10 ml) and saturated aqueous solution of sodium chloride (10 ml) in this order, and dried with anhydrous sodium sulfate. After the filtration, the filtrate was concentrated under reduced pressure, and subjected to silica gel column chromatography to produce 334 mg (42%) of the title compound as pale orange oily product from the eluate of n-hexane and ethyl acetate (1 : 1).
¹H-NMR (400 MHz, CDCl₃) δ: 1.25-1.35 (3H, m), 1.46 (9H, s), 2.22-2.22 (3H, m), 2.74-2.87 (1H, m), 3.82-3.12 (4H, m), 3.93 (2H, d, J = 3.9 Hz), 4.19-4.29 (2H, m), 4.76-4.48 (3H, m), 4.91 (1H, s), 5.84 (1/3H, s), 7.46 (1H, q, J = 6.7 Hz), 12.67 (1/3H, s).

### [Reference Example 3]

Ethyl 7-[(3S,4S)-3-tertiary butoxycarbonylamino-4-fluoromethyl-1-pyrrolidinyl]-6-fluoro-1-[(2S,1R)-2-fluorocyclopropyl]-1,4-dihydro-8-methyl-4-oxoquinoline-3-carboxylate

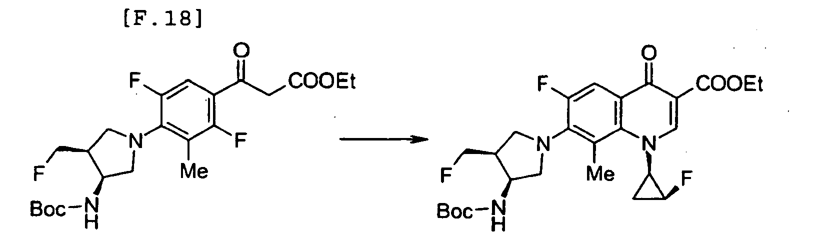

4-[(3S,4S)-3-tertiary butoxycarbonyl amino-4-fluoromethyl-1-pyrrolidinyl]-2,5-difluoro-3-methylbenzoyl acetate (334 mg, 0.729 mmol) and N,N-dimethylformamide dimethylacetal (0.194 ml, 1.46 mmol) were dissolved in benzene (6 ml), and the mixture was stirred 3 hours by heating in an oil bath at an external temperature of 80°C. The reaction solution was allowed to cool, and concentrated under reduced pressure to dryness. The resulting yellow oily product was dissolved in toluene (10 ml), and to this solution was added paratoluenesulfonate salt of (1R,2S)-2-fluorocyclopropylamine (270 mg, 1.09 mmol). The mixture was stirred at -10°C, and triethylamine (0.158 ml, 1.13 mmol) was added dropwise with stirring. The reaction solution was stirred at room temperature for 1 hour, and water (150 ml) and ethyl acetate (20 x 2 ml) were added to the solution. The solution was washed with saturated aqueous solution of sodium chloride (15 ml), and dried with anhydrous sodium sulfate. After filtration, the filtrate was concentrated under reduced pressure to dryness. The yellow oily product was dissolved in dimethylformamide (5 ml), and potassium carbonate (202 mg, 1.46 mmol) was added in an ice bath, and the solution was stirred at room temperature for 4 days. To the reaction solution was added 10% aqueous solution of citric acid (20 ml) in an ice bath, and the precipitated crystals were collected by filtration. The crystals were washed with an excess amount of purified water, and the resulting crude crystals were subjected to silica gel column chromatography to obtain 277 mg (73%) of the title compound as pale yellow powder from the eluate of a mixed solution of chloroform and methanol (95 : 5).
¹H-NMR (400 MHz, CDCl₃) δ: 1.20-1.34 (2H, m), 1.41 (3H, t, J = 7.1 Hz), 1.46 (9H, s), 2.57 (3H, s), 2.88 (1H, s), 3.14-3.18 (1H, m), 3.44-3.60 (2H, m), 3.80-3.92 (2H, m), 4.39 (2H, q, J = 7.1 Hz), 4.50-4.56 (1H, m), 4.65-4.70 (1H, m), 4.74-4.82 (1H, m), 4.94-4.90 (1H, m), 7.96 (1H, d, J = 13.2 Hz), 8.53 (1H, d, J = 2.9 Hz).

### [Example 7]

7-[(3S,4S)-3-amino-4-fluoromethyl-1-pyrrolidinyl]-6-fluoro-1-[(2S,1R)-2-(fluorocyclopropyl]-1,4-dihydro-8-methyl-4-oxoquinoline-3-carboxylic acid (Compound No. 3)

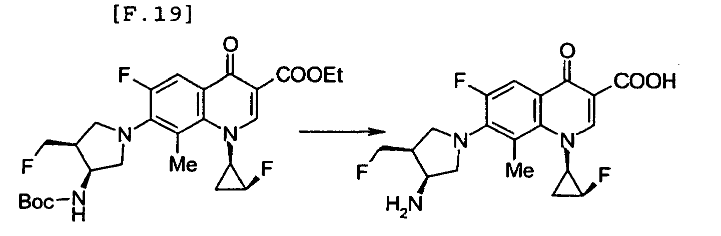

To a solution of 7-[(3S,4S)-3-tertiary butoxycarbonylamino-4-fluoromethyl-1-pyrrolidinyl]-6-fluoro-1-[(2S,1R)-2-fluorocyclopropyl]-1,4-dihydro-8-methyl-4-oxoquinoline-3-carboxylic acid in ethanol (3 ml) was added 1 mol/l aqueous solution of sodium hydroxide (1.06 ml), and the mixture was stirred at room temperature for 5 hours. To this reaction solution were added 10% aqueous solution of citric acid (15 ml) and water (10 ml) to adjust the pH to 2 to 3, and the precipitated solid was collected by filtration and washed with water (10 ml). The residue was dissolved in concentrated hydrochloric acid (5 ml) in an ice bath, and the aqueous solution was washed with chloroform (50 ml x 2). 10 mol/l aqueous solution of sodium hydroxide (6 ml) was added to the aqueous layer to adjust the pH to 12.0, and hydrochloric acid was added to this basic aqueous solution to adjust the pH to 7.4. The solution was then extracted with chloroform (100 ml x 3) and a mixed solution of chloroform and methanol (9 : 1) (100 ml x 4). The organic layer was dried with anhydrous sodium sulfate, and the solvent was distilled off under reduced pressure. The residue was purified by recrystallization from isopropyl alcohol, and dried under reduced pressure to produce 100 mg (48%) of the title compound as pale yellow crystals. The ¹H-NMR data of this compound was consistent with Compound No. 3.

### [Reference Example 5]

(3S,4S)-1-(benzyloxycarbonyl)-3-(N-tert-butoxycarbonyl-N-methyl)amino-4-fluoromethylpyrrolidine

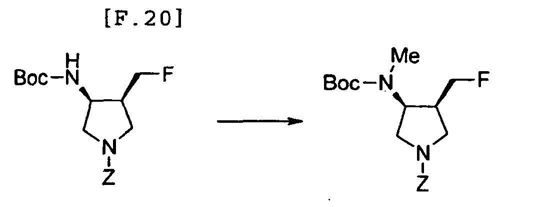

To a solution of (3S,4S)-1-(benzyloxycarbonyl)-3-(tert-butoxycarbonyl)amino-4-fluoromethylpyrrolidine (17.2 g, 48.2 mmol) in N,N-dimethylformamide (170 ml) was added sodium hydride (55%, for comparing with the 4.21 g, 96.4 mmol), and the mixture was stirred at 0°C for 10 minutes. After stirring, methyl iodide (3.30 ml, 53.0 mmol) was added to the solution at the same temperature, and the mixture was stirred for 30 minutes. A saturated aqueous solution of ammonium chloride (500 ml) was added to the reaction solution, and the solution was extracted with ethyl acetate (500 ml x 2), and washed with water (100 ml x 2) and saturated aqueous solution of sodium chloride (100 ml). The organic layer was dried with anhydrous sodium sulfate, and the solvent was distilled off under reduced pressure. The residue was purified by silica gel column chromatography (n-hexane-ethyl acetate; 4 : 1 → 2 : 1) to obtain 17.4 g (98%) of the title compound as a colorless syrup.
¹H-NMR (400 MHz, CDCl₃) δ: 1.46 (9H, d, J = 1.7 Hz), 2.78-2.80 (4H, m), 3.36-3.44 (1H, m), 3.60-3.79 (3H, m), 4.30-4.51 (1H, m), 4.58 (1H, d, J = 46.6 Hz), 4.79 (1H, brs), 5.11-5.20 (2H, m), 7.26-7.38 (5H, m).

### [Reference Example 6]

(3S,4S)-3-(N-tert-butoxycarbonyl-N-methyl)amino-4-fluoromethylpyrrolidine

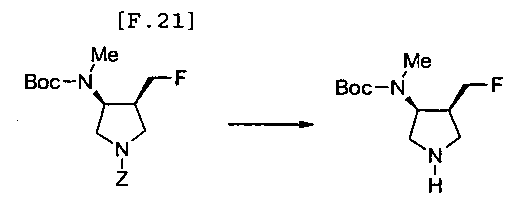

(3S,4S)-1-(benzyloxycarbonyl)-3-(N-tert-butoxycarbonyl-N-methyl)amino-4-fluoromethylpyrrolidine (15.7 g, 42.8 mmol) was dissolved in ethanol (300 ml), and 10% palladium carbon catalyst (M; water content, 50.9%; 1.60 g) was added. The mixture was stirred at 40°C for 2 hours in hydrogen stream. After removing the catalyst by filtration (by washing with ethanol), the filtrate was concentrated under reduced pressure to produce 9.50 g (96%) of the crude title compound as colorless syrup.
¹H-NMR (400 MHz, CDCl₃) δ: 1.47 (9H, s), 2.54-2.68 (1H, m), 2.85 (3H, s), 2.97 (1H, dd, J = 11.3, 7.1 Hz), 3.07 (1H, dd, J = 11.5, 5.6 Hz), 3.16-3.23 (2H, m), 4.36 (1H, ddd, J = 47.6, 9.3, 6.4 Hz), 4.48 (1H, ddd, J = 46.8, 9.1, 5.1 Hz), 4.41-4.48 (1H, m).

### [Test Example 1]

The compounds of the present invention were evaluated for their antibacterial activity according to the standard method defined by Japanese Society of Chemotherapy, and the results are shown in MIC (µg/ml) in Tables 1 and 2, below. MIC value is also shown for levofloxacin (LVFX), ciprofloxacin (CPFX), and Comparative Compound (compound of Example 1 in the Patent Document 1) for comparison with the MIC value of the compound of the present invention.

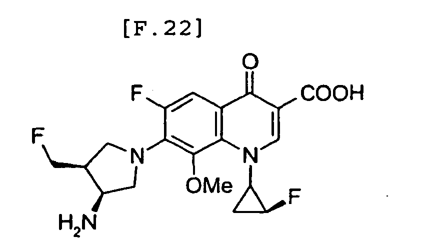

Comparative Compound

The results reveal that the compounds of the present invention have broader and stronger antibacterial activity for both Gram negative and Gram positive bacteria including the resistant bacteria compared to known synthetic quinolone antibacterials, and in particular, that the compounds of the present invention have strong antibacterial activity for Gram positive bacteria such as Staphylococcus aureus (MRSA) and pneumococcus (PRSP).

**Table 1**

| | Compound No. 1 | Compound No. 2 | Compound No. 3 | Compound No. 4 |
|---|---|---|---|---|
| E. coli, NIHJ | ≦ 0.003 | ≦ 0.003 | 0.006 | ≦ 0.003 |
| S. flexneri, 2A 5503 | ≦ 0.003 | 0.006 | ≦ 0.003 | 0.006 |
| Pr. vulgaris, 08601 | ≦ 0.003 | 0.006 | ≦ 0.003 | 0.006 |
| Pr. mirabilis, IFO-3849 | 0.012 | 0.05 | 0.006 | 0.025 |
| Ser. marcescens, 10100 | 0.025 | 0.05 | 0.025 | 0.05 |
| Ps. aeruginosa, 32104 | 0.05 | 0.1 | 0.05 | 0.1 |
| Ps. aeruginosa, 32121 | 0.025 | 0.025 | 0.025 | 0.05 |
| S. maltophilia, IID-1275 | 0.1 | 0.2 | 0.1 | 0.1 |
| S. aureus, 209P | 0.012 | 0.025 | 0.006 | 0.012 |
| S. epidermidis, 56500 | 0.025 | 0.1 | 0.025 | 0.05 |
| Str. pyogenes, G-36 | 0.1 | 0.2 | 0.05 | 0.1 |
| Str. faecalis, ATCC-19433 | 0.05 | 0.2 | 0.1 | 0.1 |
| S. aureus, 870307 | 0.2 | 0.78 | 0.39 | 0.39 |
| S. pneumoniae, J24 | 0.025 | 0.1 | 0.025 | 0.05 |
| | | | MIC values (µg/ml) | |

**Table 2**

| | Compound No. 5 | Compound No. 6 | LVFX | CPFX | Comparative Compound |
|---|---|---|---|---|---|
| E. coli, NIHJ | ≦ 0.003 | ≦ 0.003 | 0.012 | ≦ 0.003 | ≦ 0.003 |
| S. flexneri, 2A 5503 | ≦ 0.003 | ≦ 0.003 | 0.025 | 0.006 | 0.006 |
| Pr. vulgaris, 08601 | 0.006 | 0.006 | 0.012 | ≦ 0.003 | 0.012 |
| Pr. mirabilis, IFO-3849 | 0.025 | 0.012 | 0.05 | 0.012 | 0.025 |
| Ser. marcescens, 10100 | 0.05 | 0.05 | 0.1 | 0.025 | 0.05 |
| Ps. aeruginosa, 32104 | 0.2 | 0.2 | 0.2 | 0.05 | 0.2 |
| Ps. aeruginosa, 32121 | 0.05 | 0.05 | 0.1 | 0.025 | 0.05 |
| S. maltophilia, IID-1275 | 0.05 | 0.05 | 0.39 | 0.78 | 0.2 |
| S. aureus, 209P | 0.012 | 0.012 | 0.2 | 0.1 | 0.012 |
| S. epidermidis, 56500 | 0.05 | 0.025 | 0.39 | 0.2 | 0.05 |
| Str. pyogenes, G-36 | 0.1 | 0.05 | 0.78 | 1.56 | 0.1 |
| Str. faecalis, ATCC-19433 | 0.1 | 0.1 | 0.78 | 0.78 | 0.1 |
| S. aureus, 870307 | 0.39 | 0.2 | >6.25 | >6.25 | 0.2 |
| S. pneumoniae, J24 | 0.05 | 0.05 | 0.78 | 0.1 | 0.025 |
| | | | | MIC values (µg/ml) | |

| | | | | | |
|---|---|---|---|---|---|
| S. aureus, 870307: levofloxacin-resistant methicillin-resistant Staphylococcus aureus S. pneumoniae, J24: penicillin resistant pneumococcus (moderate resistance) | | | | | |

### [Test Example 2]

The compounds of the present invention were evaluated for their toxicity in a single dose toxicity test by administering the compounds to 6 week old male Slc:ddY mice. The compound was diluted with 0.1 mol/l NaOH/physiological saline, and filtered through Millex GS filter (0.22 µm) for sterilization. The compound was administered at a rate of 10 ml/kg and 0.2 ml/min in a single dose by intravenous injection. The results are shown in Table 3. The compound of Example 1 in the Patent Document 1 was used for the Comparative Compound as in the case of Test Example 1.

The results indicate that the compounds of the present invention exhibit weaker acute toxicity compared to the Comparative Compound.

**Table 3**

| | Compound No. 3 | Compound No. 4 | Comparative Compound |
|---|---|---|---|
| Dose (mg/kg) | Number of dead mice/Total | Number of dead mice/Total | Number of dead mice/Total |
| 100 | 0/5 | 0/5 | 1/5 |
| 150 | 0/3 | 1/2 | 2/3 |

## Claims

1. A compound represented by the following formula (1) : 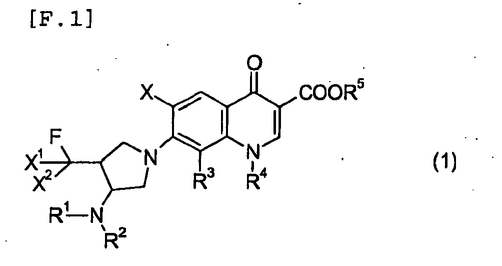 or its salt, or a hydrate thereof, wherein
R¹ represents hydrogen atom, an alkyl group containing 1 to 6 carbon atoms, a cycloalkyl group containing 3 to 6 carbon atoms, or a substituted carbonyl group derived from an amino acid, a dipeptide, or a tripeptide, wherein the alkyl group is optionally substituted with a group selected from the group consisting of hydroxy group, amino group, halogen atom, alkylthio group containing 1 to 6 carbon atoms, and alkoxy group containing 1 to 6 carbon atoms;
R² represents hydrogen atom, an alkyl group containing 1 to 6 carbon atoms, or a cycloalkyl group containing 3 to 6 carbon atoms, wherein the alkyl group is optionally substituted with a group selected from the group consisting of hydroxy group, amino group, halogen atom, alkylthio group containing 1 to 6 carbon atoms, and alkoxy group containing 1 to 6 carbon atoms;
R³ represents an alkyl group containing 1 to 6 carbon atoms;
R⁴ represents a cycloalkyl group containing 3 to 6 carbon atoms or a halogen-substituted cycloalkyl group containing 3 to 6 carbon atoms;
R⁵ represents hydrogen atom, phenyl group, acetoxymethyl group, pivaloyloxymethyl group, ethoxycarbonyl group, choline group, dimethylaminoethyl group, 5-indanyl group, phthalidyl group, 5-alkyl-2-oxo-1,3-dioxol-4-ylmethyl group, 3-acetoxy-2-oxobutyl group, an alkyl group containing 1 to 6 carbon atoms, an alkoxymethyl group containing 2 to 7 carbon atoms, or a phenylalkyl group comprising an alkylene group containing 1 to 6 carbon atoms and phenyl group;
X¹ and X² independently represent hydrogen atom or a halogen atom; and
X represents hydrogen atom or a halogen atom.

2. The compound according to claim 1, its salt, or a hydrate thereof, wherein the compound represented by formula (1) is a compound having a structure represented by the following formula: 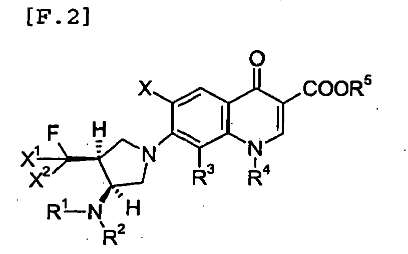 wherein R¹, R², R³, R⁴, R⁵, X¹, X², and X are as defined above.

3. The compound according to claim 1 or 2, its salt, a hydrate thereof, wherein X¹ and X² are hydrogen atom.

4. The compound according to claim 1 or 2, its salt, a hydrate thereof, wherein one of X¹ and X² is fluorine atom and the other is hydrogen atom.

5. The compound according to any one of claims 1 to 4, its salt, or a hydrate thereof, wherein R¹ and R² are hydrogen atom.

6. The compound according to any one of claims 1 to 4, its salt, or a hydrate thereof, wherein one of R¹ and R² is hydrogen atom, and the other is methyl group.

7. The compound according to any one of claims 1 to 6, its salt, or a hydrate thereof, wherein X is fluorine atom.

8. The compound according to any one of claims 1 to 6, its salt, a hydrate thereof, wherein X is hydrogen atom.

9. The compound according to any one of claims 1 to 8, its salt, or a hydrate thereof, wherein R³ is an alkyl group containing 1 to 6 carbon atoms.

10. The compound according to any one of claims 1 to 8, its salt, or a hydrate thereof, wherein R³ is methyl group.

11. The compound according to any one of claims 1 to 10, a salt thereof or a hydrate thereof, wherein R⁴ is cyclopropyl group or 1,2-cis-2-halogenocyclopropyl group.

12. The compound according to any one of claims 1 to 10, its salt, or a hydrate thereof, wherein R⁴ is (1R 2S)- or (1S, 2R)-1,2-cis-2-halogenocyclopropyl group.

13. The compound according to any one of claims 1 to 10, its salt, or a hydrate thereof, wherein R⁴ is (1R,2S)-2-halogenocyclopropyl group.

14. The compound according to any one of claims 1 to 10, its salt, or a hydrate thereof, wherein R⁴ is (1R,2S)-2-fluorocyclopropyl group.

15. The compound according to any one of claims 1 to 14, its salt, or a hydrate thereof, wherein R⁵ is hydrogen atom.

16. 7-[3-amino-4-fluoromethylpyrrolidine-1-yl]-6-fluoro-1-[(2S,1R)-2-fluorocyclopropyl]-1,4-dihydro-8-methyl-4-oxoquinoline-3-carboxylic acid, its salt, or a hydrate thereof.

17. 7-[3-fluoromethyl-4-methylaminopyrrolidine-1-yl]-6-fluoro-1-[(2S,1R)-2-fluoro-1-cyclopropyl]-1,4-dihydro-8-methyl-4-oxoquinoline-3-carboxylic acid, its salt, or a hydrate thereof.

18. 7-[cis-3-amino-4-fluoromethylpyrrolidine-1-yl]-6-fluoro-1-[(2S,1R)-2-fluorocyclopropyl]-1,4-dihydro-8-methyl-4-oxoquinoline-3-carboxylic acid, its salt, or a hydrate thereof.

19. 7-[cis-3-fluoromethyl-4-methylaminopyrrolidine-1-yl]-6-fluoro-1-[(2S,1R)-2-fluorocyclopropyl]-1,4-dihydro-8-methyl-4-oxoquinoline-3-carboxylic acid, its salt, or a hydrate thereof.

20. A compound according to any one of claim 1 to 19, its salt, or a hydrate thereof, wherein the compound of the formula (1) comprises a single diastereomer.

21. 7-[(3S,4S)-3-amino-4-fluoromethylpyrrolidine-1-yl]-6-fluoro-1-[(2S,1R)-2-fluoro-cyclopropyl]-1,4-dihydro-8-methyl-4-oxoquinoline-3-carboxylic acid, its salt, or a hydrate thereof.

22. 7-[(3S,4S)-3-fluoromethyl-4-methylaminopyrrolidine-1-yl]-6-fluoro-1-[(2S,1R)-2-fluorocyclopropyl]-1,4-dihydro-8-methyl-4-oxoquinoline-3-carboxylic acid, its salt, or a hydrate thereof.

23. A drug comprising the compound according to any one of claims 1 to 22, its salt, or a hydrate thereof, as an effective component.

24. An antibacterial drug comprising the compound according to any one of claims 1 to 22, its salt, or a hydrate thereof, as an effective component.

25. A prophylactic and/or therapeutic drug for infectious diseases, comprising the compound according to any one of claims 1 to 22, its salt, or a hydrate thereof, as an effective component.

26. A pharmaceutical composition comprising the compound according to any one of claims 1 to 22, its salt, or a hydrate thereof and a pharmaceutically acceptable carrier.

27. An antibacterial drug comprising the compound according to any one of claims 1 to 22, its salt, or a hydrate thereof and a pharmaceutically acceptable carrier.

28. A prophylactic and/or therapeutic drug comprising the compound according to any one of claims 1 to 22, its salt, or a hydrate thereof and a pharmaceutically acceptable carrier.

29. The compound according to any one of claims 1 to 22, its salt, or hydrate thereof for use in a prophylactic and/or therapeutic method.

30. The compound according to any one of claims 1 to 22, its salt, or hydrate thereof for use in a prophylactic and/or therapeutic method for an infectious disease.

31. A process for producing a drug, which comprises incorporating the compound according to any one of claims 1 to 22, its salt, or a hydrate thereof, as an effective amount therein.

32. A process for producing an antibacterial drug, which comprises incorporating the compound according to any one of claims 1 to 22, its salt, or a hydrate thereof, as an effective amount therein.

33. A process for producing a prophylactic and/or therapeutic drug, which comprises incorporating the compound according to any one of claims 1 to 22, its salt, or a hydrate thereof, as an effective amount therein.

34. Use of the compound according to any one of claims 1 to 22, its salt, or a hydrate thereof, for the production of a drug.

35. Use of the compound according to any one of claims 1 to 22, its salt, or a hydrate thereof, for the production of an antibacterial drug.

36. Use of the compound according to any one of claims 1 to 22, its salt, or a hydrate thereof, for the production of a prophylactic and/or therapeutic drug.

## Patentansprüche

1. Verbindung der folgenden Formel (1): 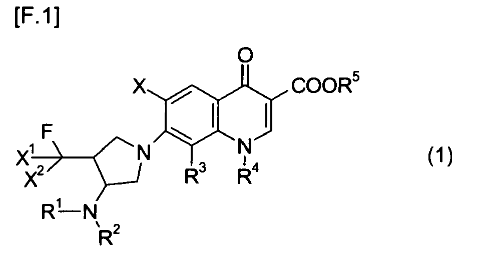 oder ihr Salz, oder ein Hydrat davon, wobei
R¹ für ein Wasserstoffatom, eine Alkylgruppe enthaltend 1 bis 6 Kohlenstoffatome, eine Cycloalkylgruppe enthaltend 3 bis 6 Kohlenstoffatome oder für eine substituierte Carbonylgruppe, die von einer Aminogruppe abgeleitet ist, für ein Dipeptid oder ein Tripeptide steht, wobei die Alkylgruppe mit einer Gruppe ausgewählt aus der Gruppe bestehend aus Hydroxygruppe, Aminogruppe, Halogenatom, Alkylthiogruppe mit 1 bis 6 Kohlenstoffatomen und Alkoxygruppe mit 1 bis 6 Kohlenstoffatomen substituiert sein kann;
R² für ein Wasserstoffatom, eine Alkylgruppe enthaltend 1 bis 6 Kohlenstoffatome oder für eine Cycloalkylgruppe enthaltend 3 bis 6 Kohlenstoffatome steht, wobei die Alkylgruppe mit einer Gruppe ausgewählt aus der Gruppe bestehend aus Hydroxygruppe, Aminogruppe, Halogenatom, Alkylthiogruppe mit 1 bis 6 Kohlenstoffatomen und Alkoxygruppe mit 1 bis 6 Kohlenstoffatomen substituiert sein kann;
R³ für eine Alkylgruppe enthaltend 1 bis 6 Kohlenstoffatome steht;
R⁴ für eine Cycloalkylgruppe enthaltend 3 bis 6 Kohlenstoffatome oder eine halogensubstituierte Cycloalkylgruppe enthaltend 3 bis 6 Kohlenstoffatome steht;
R⁵ für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminoethylgruppe, eine 5-Indanylgruppe, eine Phthalidylgruppe, eine 5-Alkyl-2-oxo-1,3-dioxol-4-ylmethylgruppe, eine 3-Acetoxy-2-oxobutylgruppe, eine Alkylgruppe enthaltend 1 bis 6 Kohlenstoffatome, eine Alkoxymethylgruppe enthaltend 2 bis 7 Kohlenstoffatome oder für eine Phenylalkylgruppe umfassend eine Alkylengruppe enthaltend 1 bis 6 Kohlenstoffatome und eine Phenylgruppe steht;
X¹ und X² unabhängig voneinander für ein Wasserstoffatom oder ein Halogenatom stehen; und
X für ein Wasserstoffatom oder ein Halogenatom steht.

2. Die Verbindung gemäß Anspruch 1, ihr Salz oder ein Hydrat davon, worin die Verbindung der Formel (1) eine Verbindung mit einer Strukur der folgenden Formel ist: 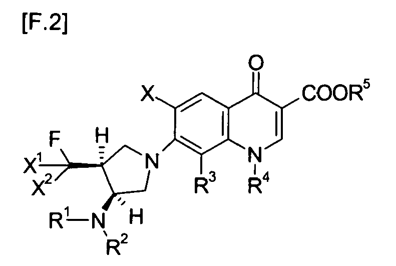 worin R¹, R², R³, R⁴, R⁵, X¹, X², und X wie oben definiert sind.

3. Die Verbindung gemäß Anspruch 1 oder 2, ihr Salz oder ein Hydrat davon, worin X¹ und X² Wasserstoffatome sind.

4. Die Verbindung gemäß Anspruch 1 oder 2, ihr Salz oder ein Hydrat davon, worin eines von X¹ und X² ein Fluoratom und das andere ein Wasserstoffatom ist.

5. Die Verbindung gemäß einem der Ansprüche 1 bis 4, ihr Salz oder ein Hydrat davon, worin R¹ and R² Wasserstoffatome sind.

6. Die Verbindung gemäß einem der Ansprüche 1 bis 4, ihr Salz oder ein Hydrat davon, worin eines von R¹ und R² ein Wasserstoffatom und das andere eine Methylgruppe ist.

7. Die Verbindung gemäß einem der Ansprüche 1 bis 6, ihr Salz oder ein Hydrat davon, worin X ein Fluoratom ist.

8. Die Verbindung gemäß einem der Ansprüche 1 bis 6, ihr Salz oder ein Hydrat davon, worin X ein Wasserstoffatom ist.

9. Die Verbindung gemäß einem der Ansprüche 1 bis 8, ihr Salz oder ein Hydrat davon, worin R³ eine Alkylgruppe enthaltend 1 bis 6 Kohlenstoffatome ist.

10. Die Verbindung gemäß einem der Ansprüche 1 bis 8, ihr Salz oder ein Hydrat davon, worin R³ eine Methylgruppe ist.

11. Die Verbindung gemäß einem der Ansprüche 1 bis 10, ihr Salz oder ein Hydrat davon, worin R⁴ eine Cyclopropylgruppe oder eine 1,2-cis-2-Halogencyclopropylgruppe ist.

12. Die Verbindung gemäß einem der Ansprüche 1 bis 10, ihr Salz oder ein Hydrat davon, worin R⁴ eine (1R,2S)- oder (1S,2R)-1,2-cis-2-Halogencyclopropylgruppe ist.

13. Die Verbindung gemäß einem der Ansprüche 1 bis 10, ihr Salz oder ein Hydrat davon, worin R⁴ eine (1R,2S)-2-Halogencyclopropylgruppe ist.

14. Die Verbindung gemäß einem der Ansprüche 1 bis 10, ihr Salz oder ein Hydrat davon, worin R⁴ eine (1R,2S)-2-Fluorcyclopropylgruppe ist.

15. Die Verbindung gemäß einem der Ansprüche 1 bis 14, ihr Salz oder ein Hydrat davon, worin R⁵ ein Wasserstoffatom ist.

16. 7-[3-Amino-4-fluormethylpyrrolidin-1-yl]-6-fluor-1-[(2S,1R)-2-fluorcyclopropyl]-1,4-dihydro-8-methyl-4-oxochinolin-3-carbonsäure, ihr Salz oder ein Hydrat davon.

17. 7-[3-Fluormethyl-4-methylaminopyrrolidin-1-yl]-6-fluor-1-[(2S,1R)-2-fluor-1-cyclopropyl]-1,4-dihydro-8-methyl-4-oxochinolin-3-carbonsäure, ihr Salz oder ein Hydrat davon.

18. 7-[cis-3-Amino-4-fluormethylpyrrolidin-1-yl]-6-ftuor-1-[(2S,1R)-2-fluorcyclopropyl]-1,4-dihydro-8-methyl-4-oxochinolin-3-carbonsäure, ihr Salz oder ein Hydrat davon.

19. 7-[cis-3-Fluormethyl-4-methylaminopyrrolidin-1 -yl]-6-fluor-1-[(2S,1R)-2-fluorcyclopropyl]-1,4-dihydro-8-methyl-4-oxochinolin-3-carbonsäure, ihr Salz ode rein Hydrat davon.

20. Verbindung gemäß einem der Ansprüche 1 bis 19, ihr Salz oder rein Hydrat davon, wobei die Verbindung der Formel (1) ein einziges Diastereomer enthält.

21. 7-[(3S,4S)-3-Amino-4-fluormethylpyrrolidin-1-yl]-6-fluor-1-[(2S,1R)-2-fluor-cyclopropyl]-1,4-dihydro-8-methyl-4-oxochinolin-3-carbonsäure, ihr Salz, ode rein Hydrat davon.

22. 7-[(3S,4S)-3-Fluormethyl-4-methylaminopyrrolidin-1-yl]-6-fluor-1-[(2S,1R)-2-fluorcyclopropyl]-1,4-dihydro-8-methyl-4-oxochinolin-3-carbonsäure, ihr Salz oder ein Hydrat davon.

23. Arzneimittel umfassend die Verbindung gemäß einem der Ansprüche 1 bis 22, ihr Salz oder ein Hydrat davon als aktive Komponente.

24. Antibakterielles Arzneimittel umfassend die Verbindung gemäß einem der Ansprüche 1 bis 22, ihr Salz oder ein Hydrat davon als aktive Komponente.

25. Prophylaktisches und/oder therapeutisches Arzneimittel für Infektionskrankheiten, umfassend die Verbindung gemäß einem der Ansprüche 1 bis 22, ihr Salz oder ein Hydrat davon als aktive Komponente.

26. Pharmazeutische Zusammensetzung enthaltend die Verbindung gemäß einem der Ansprüche 1 bis 22, ihr Salz oder ein Hydrat davon und einen pharmazeutisch verträglichen Träger.

27. Antibakterieller Arzneistoff enthaltend die Verbindung gemäß einem der Ansprüche 1 bis 22, ihr Salz oder ein Hydrat davon und einen pharmazeutisch verträglichen Träger.

28. Prophylaktisches und/oder therapeutisches Arzneimittel, umfassend die Verbindung gemäß einem der Ansprüche 1 bis 22, ihr Salz oder ein Hydrat davon und einen pharmazeutisch verträglichen Träger.

29. Die Verbindung gemäß einem der Ansprüche 1 bis 22, ihr Salz oder ein Hydrat davon zur Verwendung in einem prophylaktischen und/oder therapeutischen Verfahren.

30. Die Verbindung gemäß einem der Ansprüche 1 bis 22, ihr Salz oder ein Hydrat davon zur Verwendung in einem prophylaktischen und/oder therapeutischen Verfahren bei einer Infektionskrankheit.

31. Verfahren zur Herstellung eines Arzneimittels, umfassend Einfügen der Verbindung gemäß einem der Ansprüche 1 bis 22, ihres Salzes oder eines Hydrats davon in das Arzneimittel in einer wirksamen Menge.

32. Verfahren zur Herstellung eines antibakteriellen Arzneimittels, umfassend Einfügen der Verbindung gemäß einem der Ansprüche 1 bis 22, ihres Salz oder eines Hydrats davon in das Arzneimittel in einer wirksamen Menge.

33. Verfahren zur Herstellung eines prophylaktischen und/oder therapeutischen Arzneimittels, umfassend Einfügen der Verbindung gemäß einem der Ansprüche 1 bis 22, ihres Salzes oder eines Hydrats davon in das Arzneimittel in einer wirksamen Menge.

34. Verwendung der Verbindung gemäß einem der Ansprüche 1 bis 22, ihres Salzes oder eines Hydrats davon zur Herstellung eines Arzneimittels.

35. Verwendung der Verbindung gemäß einem der Ansprüche 1 bis 22, ihres Salzes oder eines Hydrats davon zur Herstellung eines antibakteriellen Arzneimittels.

36. Verwendung der Verbindung gemäß einem der Ansprüche 1 bis 22, ihres Salzes oder eines Hydrats davon zur Herstellung eines prophylaktischen und/oder therapeutischen Arzneimittels.

## Revendications

1. Composé représenté par la formule (1) suivante: 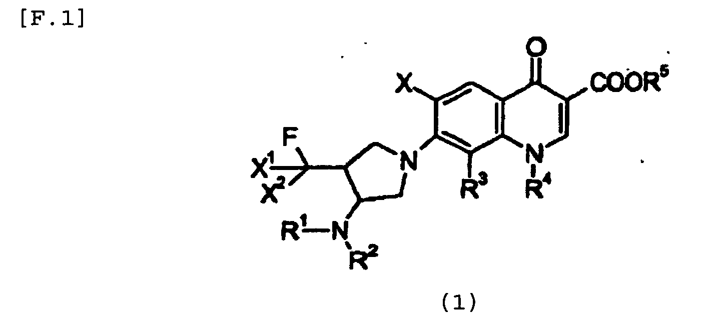 ou son sel, ou un hydrate de celui-ci,
formule dans laquelle
R¹ représente un atome d'hydrogène, un groupe alkyle contenant 1 à 6 atomes de carbone, un groupe cycloalkyle contenant 3 à 6 atomes de carbone, ou un groupe carbonyle substitué dérivé d'un acide aminé, d'un dipeptide ou d'un tripeptide, le groupe alkyle étant éventuellement substitué par un groupe choisi dans l'ensemble constitué par un groupe hydroxy, un groupe amino, un atome d'halogène, un groupe alkylthio contenant 1 à 6 atomes de carbone, et un groupe alcoxy contenant 1 à 6 atomes de carbone ;
R² représente un atome d'hydrogène, un groupe alkyle contenant 1 à 6 atomes de carbone, ou un groupe cycloalkyle contenant 3 à 6 atomes de carbone, le groupe alkyle étant éventuellement substitué par un groupe choisi dans l'ensemble constitué par un groupe hydroxy, un groupe amino, un atome d'halogène, un groupe alkylthio contenant 1 à 6 atomes de carbone, et un groupe alcoxy contenant 1 à 6 atomes de carbone ;
_{R}³ représente un groupe alkyle contenant 1 à 6 atomes de carbone ;
R⁴ représente un groupe cycloalkyle contenant 3 à 6 atomes de carbone ou un groupe cycloalkyle halogéno-substitué contenant 3 à 6 atomes de carbone ;
R⁵ représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidyle, un groupe 5-alkyl-2-oxo-1,3-dioxol-4-ylméthyle, un groupe 3-acétoxy-2-oxobutyle, un groupe alkyle contenant 1 à 6 atomes de carbone, un groupe alcoxyméthyle contenant 2 à 7 atomes de carbone, ou un groupe phénylalkyle comprenant un groupe alkylène contenant 1 à 6 atomes de carbone et un groupe phényle ;
X¹ et X² représentent indépendamment un atome d'hydrogène ou un atome d'halogène ; et
X représente un atome d'hydrogène ou un atome d'halogène.

2. Composé selon la revendication 1, son sel, ou un hydrate de celui-ci, dans lequel le composé représenté par la formule (1) est un composé ayant une structure représentée par la formule suivante: 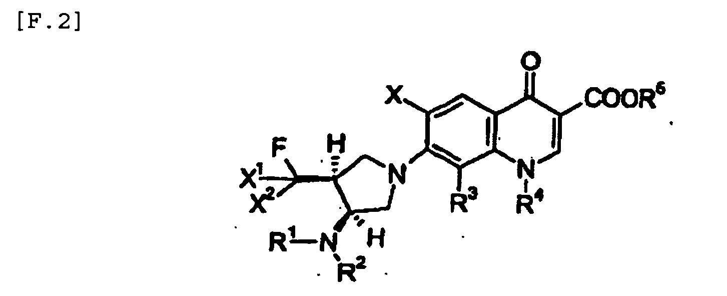 dans laquelle R¹, R², R³, R⁴, R⁵, X¹, X² et X sont tels que définis ci-dessus.

3. Composé selon la revendication 1 ou 2, son sel, ou un hydrate de celui-ci, dans lequel X¹ et X² sont des atomes d'hydrogène.

4. Composé selon la revendication 1 ou 2, son sel, ou un hydrate de celui-ci, dans lequel l'un de X¹ et X² est un atome de fluor et l'autre est un atome d'hydrogène.

5. Composé selon l'une quelconque des revendications 1 à 4, son sel, ou un hydrate de celui-ci, dans lequel R¹ et R² sont des atomes d'hydrogène.

6. Composé selon l'une quelconque des revendications 1 à 4, son sel, ou un hydrate de celui-ci, dans lequel l'un de R¹ et R² est un atome d'hydrogène et l'autre est un groupe méthyle.

7. Composé selon l'une quelconque des revendications 1 à 6, son sel, ou un hydrate de celui-ci, dans lequel X est un atome de fluor.

8. Composé selon l'une quelconque des revendications 1 à 6, son sel, ou un hydrate de celui-ci, dans lequel X est un atome d'hydrogène.

9. Composé selon l'une quelconque des revendications 1 à 8, son sel, ou un hydrate de celui-ci, dans lequel R³ est un groupe alkyle contenant 1 à 6 atomes de carbone.

10. Composé selon l'une quelconque des revendications 1 à 8, son sel, ou un hydrate de celui-ci, dans lequel R³ est un groupe méthyle.

11. Composé selon l'une quelconque des revendications 1 à 10, son sel, ou un hydrate de celui-ci, dans lequel R⁴ est un groupe cyclopropyle ou un groupe 1,2-cis-2-halogénocyclopropyle.

12. Composé selon l'une quelconque des revendications 1 à 10, son sel, ou un hydrate de celui-ci, dans lequel R⁴ est un groupe (1R,2S)- ou (1S,2R)-1,2-cis-2-halogénocyclopropyle.

13. Composé selon l'une quelconque des revendications 1 à 10, son sel, ou un hydrate de celui-ci, dans lequel R⁴ est un groupe (1R,2S)-2-halogénocyclopropyle.

14. Composé selon l'une quelconque des revendications 1 à 10, son sel, ou un hydrate de celui-ci, dans lequel R⁴ est un groupe (1R,2S)-2-fluorocyclopropyle.

15. Composé selon l'une quelconque des revendications 1 à 14, son sel, ou un hydrate de celui-ci, dans lequel R⁵ est un atome d'hydrogène.

16. Acide 7-[3-amino-4-fluorométhylpyrrolidin-1-yl]-6-fluoro-1-[(2S,1R)-2-fluorocyclopropyl]-1,4-dihydro-8-méthyl-4-oxoquinoline-3-carboxylique, son sel, ou un hydrate de celui-ci.

17. Acide 7-[3-fluorométhyl-4-méthylaminopyrrolidin-1-yl]-6-fluoro-1-[(2S,1R)-2-fluoro-1-cyclopropyl]-1,4-dihydro-8-méthyl-4-oxoquinoline-3-carboxylique, son sel, ou un hydrate de celui-ci.

18. Acide 7-[cis-3-amino-4-fluorométhylpyrrolidin-1-yl]-6-fluoro-1-[(2S,1R)-2-fluorocyclopropyl]-1,4-dihydro-8-méthyl-4-oxoquinoline-3-carboxylique, son sel, ou un hydrate de celui-ci.

19. Acide 7-[cis-3-fluorométhyl-4-méthylamino-pyrrolidin-1-yl]-6-fluoro-1-[(2S,1R)-2-fluorocyclopropyl]-1,4-dihydro-8-méthyl-4-oxoquinoline-3-carboxylique, son sel, ou un hydrate de celui-ci.

20. Composé selon l'une quelconque des revendications 1 à 19, son sel, ou un hydrate de celui-ci, dans lequel le composé de formule (1) comprend un seul diastéréomère.

21. Acide 7-[(3S,4S)-3-amino-4-fluorométhyl-pyrrolidin-1-yl]-6-fluoro-1-[(2S,1R)-2-fluorocyclopropyl]-1,4-dihydro-8-méthyl-4-oxoquinoline-3-carboxylique, son sel, ou un hydrate de celui-ci.

22. Acide 7-[(3S,4S)-3-fluorométhyl-4-méthylamino-pyrrolidin-1-yl]-6-fluoro-1-[(2S,1R)-2-fluorocyclopropyl]-1,4-dihydro-8-méthyl-4-oxoquinoline-3-carboxylique, son sel, ou un hydrate de celui-ci.

23. Médicament comprenant le composé selon l'une quelconque des revendications 1 à 22, son sel, ou un hydrate de celui-ci, à titre d'agent actif.

24. Médicament antibactérien comprenant le composé selon l'une quelconque des revendications 1 à 22, son sel, ou un hydrate de celui-ci, à titre d'agent actif.

25. Médicament prophylactique et/ou thérapeutique contre des maladies infectieuses, comprenant le composé selon l'une quelconque des revendications 1 à 22, son sel, ou un hydrate de celui-ci, à titre d'agent actif.

26. Composition pharmaceutique comprenant le composé selon l'une quelconque des revendications 1 à 22, son sel, ou un hydrate de celui-ci, et un véhicule pharmaceutiquement acceptable.

27. Médicament antibactérien comprenant le composé selon l'une quelconque des revendications 1 à 22, son sel, ou un hydrate de celui-ci, et un véhicule pharmaceutiquement acceptable.

28. Médicament prophylactique et/ou thérapeutique comprenant le composé selon l'une quelconque des revendications 1 à 22, son sel, ou un hydrate de celui-ci, et un véhicule pharmaceutiquement acceptable.

29. Composé selon l'une quelconque des revendications 1 à 22, son sel, ou un hydrate de celui-ci, pour utilisation dans une méthode prophylactique et/ou thérapeutique.

30. Composé selon l'une quelconque des revendications 1 à 22, son sel, ou un hydrate de celui-ci, pour utilisation dans une méthode prophylactique et/ou thérapeutique contre une maladie infectieuse.

31. Procédé pour produire un médicament, qui comprend l'incorporation dans celui-ci, sous la forme d'une quantité efficace, du composé selon l'une quelconque des revendications 1 à 22, de son sel, ou d'un hydrate de celui-ci.

32. Procédé pour produire un médicament antibactérien, qui comprend l'incorporation dans celui-ci, sous la forme d'une quantité efficace, du composé selon
l'une quelconque des revendications 1 à 22, de son sel, ou d'un hydrate de celui-ci.

33. Procédé pour produire un médicament prophylactique et/ou thérapeutique, qui comprend l'incorporation dans celui-ci, sous la forme d'une quantité efficace, du composé selon l'une quelconque des revendications 1 à 22, de son sel, ou d'un hydrate de celui-ci.

34. Utilisation du composé selon l'une quelconque des revendications 1 à 22, de son sel, ou d'un hydrate de celui-ci, pour la production d'un médicament.

35. Utilisation du composé selon l'une quelconque des revendications 1 à 22, de son sel, ou d'un hydrate de celui-ci, pour la production d'un médicament antibactérien.

36. Utilisation du composé selon l'une quelconque des revendications 1 à 22, de son sel, ou d'un hydrate de celui-ci, pour la production d'un médicament prophylactique et/ou thérapeutique.
